# EUROPEAN PATENT SPECIFICATION

(11) **EP 3 880 847 B1**
(45) Date of publication and mention of the grant of the patent: **03.09.2025**
(21) Application number: 19861243.4
(22) Date of filing: 15.11.2019
(51) Int. Cl.: C12Q 1/6886

(54) **METHODS AND COMPOSITIONS FOR CHARACTERIZING BLADDER CANCER**
VERFAHREN UND ZUSAMMENSETZUNGEN ZUR CHARAKTERISIERUNG VON BLASENKREBS
PROCÉDÉS ET COMPOSITIONS DE CARACTÉRISATION DU CANCER DE LA VESSIE

(30) Priority: 16.11.2018 US 201862768538 P
(43) Date of publication of application: 22.09.2021
(73) Proprietor: Oslo Universitetssykehus HF, 0424 Oslo (NO)
(72) Inventor: LIND, Guro, E., 0424 Oslo (NO); WAHLQVIST, Rolf, 0424 Oslo (NO); LOTHE, Ragnhild, A., 0424 Oslo (NO); JERONIMO, Carmen, 0424 Oslo (NO); PHARO, Heidi, D., 0424 Oslo (NO)
(74) Representative: Algemeen Octrooi- en Merkenbureau B.V.
(86) International application number: PCT/IB2019/001441
(87) International publication number: WO 2020/099938

(56) References cited:
- WO-A1-2014/183093
- WO-A1-2016/205236
- WO-A1-2016/207656
- WO-A2-2010/099489
- WO-A9-2014/183093
- US-A1- 2010 317 000
- US-A1- 2013 224 738
- SOJUN HOSHIMOTO: "AIM1 and LINE-1 Epigenetic Aberrations in Tumor and Serum Relate to Melanoma Progression and Disease Outcome - Supplementary Figures", 1 June 2012 (2012-06-01), XP055686064, Retrieved from the Internet <URL:https://ars.els-cdn.com/content/image/1-s2.0-S0022202X15357730-mmc1.pdf> [retrieved on 20200415]
- HEIDIDIETRICHSON PHARO ET AL: "BladMetrix: a novel urine DNA methylation test with high accuracy for detection of bladder cancer in hematuria patients", CLINICAL EPIGENETICS, BIOMED CENTRAL LTD, LONDON, UK, vol. 14, no. 1, 17 September 2022 (2022-09-17), pages 1 - 9, XP021308046, ISSN: 1868-7075, DOI: 10.1186/S13148-022-01335-2
- DIETRICHSON PHARO HEIDI: "BladMetrix: a novel urine DNA methylation test with high accuracy for detection of bladder cancer in hematuria patients - SUPPLEMENTARY DATA.", CLINICAL EPIGENETICS, 17 September 2022 (2022-09-17), XP093038042, Retrieved from the Internet <URL:https://clinicalepigeneticsjournal.biomedcentral.com/articles/10.1186/s13148-022-01335-2> [retrieved on 20230406]
- SOJUN HOSHIMOTO ET AL: "AIM1 and LINE-1 Epigenetic Aberrations in Tumor and Serum Relate to Melanoma Progression and Disease Outcome", JOURNAL OF INVESTIGATIVE DERMATOLOGY, vol. 132, no. 6, 1 June 2012 (2012-06-01), NL, pages 1689 - 1697, XP055686053, ISSN: 0022-202X, DOI: 10.1038/jid.2012.36
- THOMAS REINERT ET AL: "Diagnosis of Bladder Cancer Recurrence Based on Urinary Levels of EOMES, HOXA9, POU4F2, TWIST1, VIM, and ZNF154 Hypermethylation", PLOS ONE, vol. 7, no. 10, 3 October 2012 (2012-10-03), pages e46297, XP055295205, DOI: 10.1371/journal.pone.0046297
- BRAIT MARIANA ET AL: "Aberrant promoter methylation of multiple genes during pathogenesis of bladder cancer", CANCER EPIDEMIOLOGY, BIOMARKERS AND PREVENTION, PHILADELPHIA, PA, US, vol. 17, no. 10, 1 October 2008 (2008-10-01), pages 2786 - 2794, XP002585336, ISSN: 1055-9965, DOI: 10.1158/1055-9965.EPI-08-0192
- MARIANA BRAIT: "Aberrant promoter methylation of multiple genes during pathogenesis of bladder cancer - Supplementary Data", 1 October 2008 (2008-10-01), XP055686066, Retrieved from the Internet <URL:https://cebp.aacrjournals.org/content/cebp/suppl/2008/10/15/17.10.2786.DC1/0192_Supplementary_Tables_1-2.pdf> [retrieved on 20200415]

## Description

### FIELD OF THE INVENTION

Provided herein are compositions and methods for characterizing bladder cancer. In particular, provided herein are methylation markers for bladder cancer and bladder cancer recurrence.

### BACKGROUND OF THE INVENTION

Bladder cancer is the ninth most common type of cancer worldwide, accounting for around 430,000 new cases and over 160,000 deaths each year¹. Initial symptoms are typically unspecific and include hematuria and irritative voiding symptoms. Most patients (~75%) present with non-muscle-invasive bladder cancer (NMIBC) and are initially given a good prognosis², but as many as 50-70% experience recurrence and up to 20% progress to invasive disease³. The remaining patients (~25%) are diagnosed with muscle-invasive bladder cancer (MIBC) and have an estimated survival rate of only 35%⁴. The gold standard for both detection and surveillance of bladder cancer is cystoscopy, *i.e.* an endoscopic examination of the urinary mucous membrane. The frequent use of this invasive and painful procedure contributes to make bladder cancer one of the most expensive cancers to manage⁵. Cystoscopy is also known to be operator-dependent and the diagnostic accuracy is suboptimal⁶. In a meta-analysis, white-light cystoscopy was reported to have an average sensitivity and specificity of 71% (49-93%) and 72% (47-96%), respectively⁷. The detection rate is particularly poor for early stage tumors (Ta and T1) and high grade carcinomas *in situ*^{8,9}. Altogether, there is a clear need for a non-invasive, accurate and less expensive test for detection of bladder tumors.

Urine-based tests represent a promising non-invasive alternative to cystoscopy, as the great majority of bladder cancers (90-95%) arise in the urothelium and shed cells directly into the urine flow^{10,11}. Urine cytology, *i.e.* a visual inspection of the cells in the urine, is typically used in combination with cystoscopy, but suffers from poor sensitivity, especially for low-grade tumors¹². Six other urine-based tests have so far obtained FDA approval, with sensitivities ranging from 58% to 78% and specificities from 74% to 88%¹³. Moreover, a variety of urinary molecular biomarkers have been reported in the literature¹⁴⁻¹⁸, but none have so far managed to outperform cystoscopy. Typical study limitations include use of small and/or unrepresentative study cohorts and lack of validation.

Aberrant DNA methylation is a frequent and often early event in the bladder carcinogenesis, and has promising potential as a cancer biomarker¹⁹. The methylation mark is known to be present in various bodily fluids, and can be detected by well-established techniques such as PCR-based method²⁰. Traditionally, search for DNA methylation biomarkers was restricted to the promoters of annotated genes with functional relevance²¹. Advances in both high-throughput sequencing technology and bioinformatic analyses have enabled comprehensive DNA methylation analyses, *i.e.* across the entire methylome, independent of annotation and functionality. Reduced representation bisulfite sequencing (RRBS)²² is a methylome sequencing approach that captures the majority of promoters and other relevant genomic regions, reducing the amount of sequencing required to obtain good coverage of important genomic features. With the concomitant development of the digital PCR technology, recently optimized for robust DNA methylation analyses²³, detection and precise quantification of biomarkers present in low quantities in liquid biopsies, e.g. urine, has been largely facilitated²⁴. WO2016205236A1 involves methods for detecting and diagnosing, urothelial cancers by detecting methylation changes in the regulatory region of specific nucleic acid sequences in a sample from a subject. A systematic review of the diagnostic accuracy of methylation markers in urine for the detection of bladder cancer was recently performed by Bosschieter et al¹⁵.

Additional markers for non-invasive detection of bladder cancer are needed.

### SUMMARY OF THE INVENTION

Provided herein are compositions and methods for characterizing bladder cancer. In particular, provided herein are methylation markers for bladder cancer and bladder cancer recurrence.

Experiments described herein used a methylome sequencing approach to identify bladder cancer-specific biomarkers. The best performing biomarkers were validated in a series of urine samples using targeted digital PCR analysis. A combination of the three best performing markers discriminated bladder cancers from healthy controls with a high accuracy (96% sensitivity; 100% specificity). The biomarkers also exhibited high specificity (80%) for discriminating between bladder cancer and other urological cancer types, such as prostate and kidney. These results provide an effective non-invasive test for detection of bladder cancer, useful both for initial diagnosis and monitoring of surgically treated patients for disease recurrence, reducing the need for cystoscopy.

For example, in some embodiments, provided herein is a method of identifying methylation markers in a sample, comprising: detecting the presence of aberrant methylation in one or more (e.g., 1, 2, 3, 4, 5, 6, or 7) nucleic acids selected from, for example, SEQ ID NOs: 1-7 or 15-21, in a sample from a subject. The present invention relates to a method of identifying methylation markers in a sample, comprising: detecting the presence of aberrant methylation in nucleic acid SEQ ID NO: 1, and one or more nucleic acids selected from the group consisting of SEQ ID NOs: 2-7 in a sample from a subject. In some embodiments, the sample is urine. In some embodiments, the subject has previously been treated for bladder cancer or is suspected of having bladder cancer. In some embodiments, urine samples are collected before diagnosis from patients suspected of having bladder cancer. In some embodiments, the patient suspected of having bladder cancer presents with hematuria. In some embodiments, the patient monitored for recurrence has previously been diagnosed with and treated for non-muscle invasive bladder cancer. In some embodiments, the patient monitored for recurrence has been treated with trans-urethreal transection of bladder tumor (TURBT). In some embodiments, the urine samples are collected at different intervals after treatment. In some embodiments, methylation levels of one or more of the methylation markers are compared before and after treatment. In some embodiments, decrease in the methylation signal of the methylation markers relative to pre-treatment levels indicates successful treatment. In some embodiments, an increase in the levels of at least one of the methylation markers after the first post-treatment monitoring sample indicates a disease recurrence.

The present disclosure is not limited to a particular method of detecting the presence of aberrant methylation. Examples include, but are not limited to, methylation specific PCR, methylated DNA immunoprecipitation (MeDIP), or pyrosequencing of bisulfite treated DNA. In some embodiments, the aberrant methylation is a change in methylation levels or patterns relative to a control nucleic acid (e.g., unmethylated nucleic or a nucleic acid from a subject not diagnosed with bladder cancer). In some embodiments, the method further comprises detecting the presence of aberrant methylation in a VIM nucleic acid. In some embodiments, the detecting comprises the steps of a) modifying said nucleic acid with bisulfite; and b) detecting the bisulfite modified nucleic acid using an amplification method.

In some embodiments not part of the invention, the method further comprises the step of treating the subject for bladder cancer (e.g., recurrent or metastatic bladder cancer) when the aberrant methylation is identified. The present disclosure is not limited to particular treatments for bladder cancer. Examples include, but are not limited to, chemotherapy, radiation or surgery.

Further embodiments not part of the invention provide a method for treating recurrent bladder cancer in a subject, comprising: a) detecting the presence of aberrant methylation in one or more (e.g., 1, 2, 3, 4, 5, 6, or 7) nucleic acids selected from, for example, SEQ ID NOs: 1-7 in a urine sample from a subject previously treated for bladder cancer; b) identifying a subject with aberrant methylation; and c) administering a treatment for recurrent bladder cancer when the subject is identified.

Yet other embodiments not part of the invention provide a method for treating a subject having or suspected of having recurrent bladder cancer, comprising: a) performing or having performed an assay on urine sample to identify the subject as having the presence of aberrant methylation in one or more nucleic acids selected from, for example, SEQ ID NOs: 1-7; b) identifying whether or not the subject has the presence of aberrant methylation in one or more nucleic acids selected from, for example, SEQ ID NOs: 1-7 in a urine sample from said subject; and c) treating the subject for recurrent bladder cancer when the presence of the aberrant methylation is detected.

Certain embodiments provide a method of diagnosing bladder cancer, comprising: a) identifying the presence of aberrant methylation in a nucleic acid selected from, for example, SEQ ID NOs: 1-7 in a sample from a subject; and b) diagnosing bladder cancer in the subject when the methylation is present.

Additional embodiments provide a kit, comprising: a plurality of nucleic acid reagents that detect the presence of aberrant methylation in one or more (e.g., 1, 2, 3, 4, 5, 6, or 7) nucleic acids selected from, for example, SEQ ID NOs: 1-7. The present invention relates a kit, comprising: a plurality of nucleic acid reagents that detect the presence of aberrant methylation in nucleic acid SEQ ID NO: 1, and one or more nucleic acids selected from the group consisting of SEQ ID NOs: 2-7.In some embodiments, the reagents are nucleic acid primers or probes that bind to bisulfite modified nucleic acids but not unmodified nucleic acids. In some embodiments, the reagents bind to one or more nucleic acids selected from, for example, of SEQ ID NOs: 8-14. In some embodiments, the primers or probes are labeled. In some embodiments, the kit further comprises one or more reagents for performing a methylation specific detection assay (e.g., including but not limited to, bisulfite, buffers, detection reagents, etc.).

Additional embodiments will be apparent to persons skilled in the relevant art based on the teachings contained herein.

### DESCRIPTION OF THE DRAWINGS

FIG. 1: Overview of the different steps in the in-house developed bioinformatic pipeline for identification of differentially methylated regions.
FIG. 2: RRBS mapping efficiency of urological cancer cell lines. On average, 71% of the quality controlled and trimmed reads were uniquely mapped to the bisulfite treated genome (hg19). 15.0% of the reads had multiple hits, and 14.1% were unmapped reads.
FIG. 3: Example of a bladder cancer biomarker candidate identified from methylome sequencing. A) A representative biomarker candidate on chromosome 16 is shown, including both 1000 bp windows (red boxes; n=6) and 200 bp windows (orange boxes; n=3) that overlap by 100 bp. B) A more detailed view of one of the 200 bp windows (i.e. marker 16-89314201in Table 1-3) from A) is shown.
FIG. 4: Stepwise selection of DNA methylation biomarkers based on an initial list of 32 candidates identified from methylome sequencing. Abbreviations: Ct, threshold cycle; ddPCR; droplet digital PCR; NTC, non-template control; qMSP, quantitative methylation-specific PCR; RRBS, reduced representation bisulfite sequencing; SD-curve, standard curve.
FIG. 5: The performance of the top eight biomarkers in detecting bladder cancer patients. Each row represent one bladder cancer patient and each column represent one biomarker. "FALSE" indicate that the biomarker is scored unmethylated for the respective patient, and "TRUE" means that it is scored methylated. The rightmost column summarizes the number of biomarkers that are scored methylated per patient. The two last rows show the number of patients that are scored methylated, and the sensitivity of each biomarker, respectively.
FIG. 6: Receiver operator characteristic (ROC) curves showing the performance of the methylation biomarkers for the detection of bladder cancer in urine. Diagnostic performance of the 5-marker panel for detection of bladder cancer patients is shown.
FIG. 7: Biomarker performance in the monitoring setting. The four plots represent four different patients. The normalized methylation value is indicated on the y-axis, and the time points of cystoscopy and urine sampling at the x-axis. Each colored line represent one biomarker (n=8).

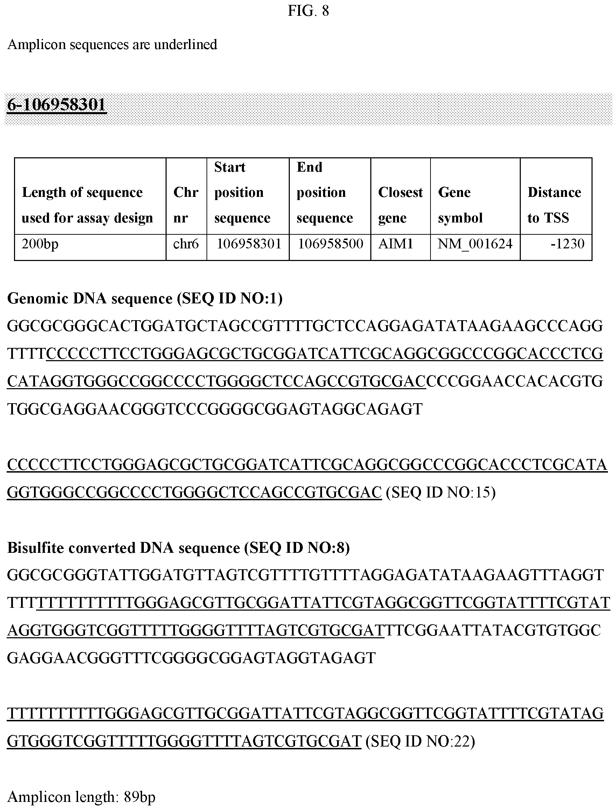

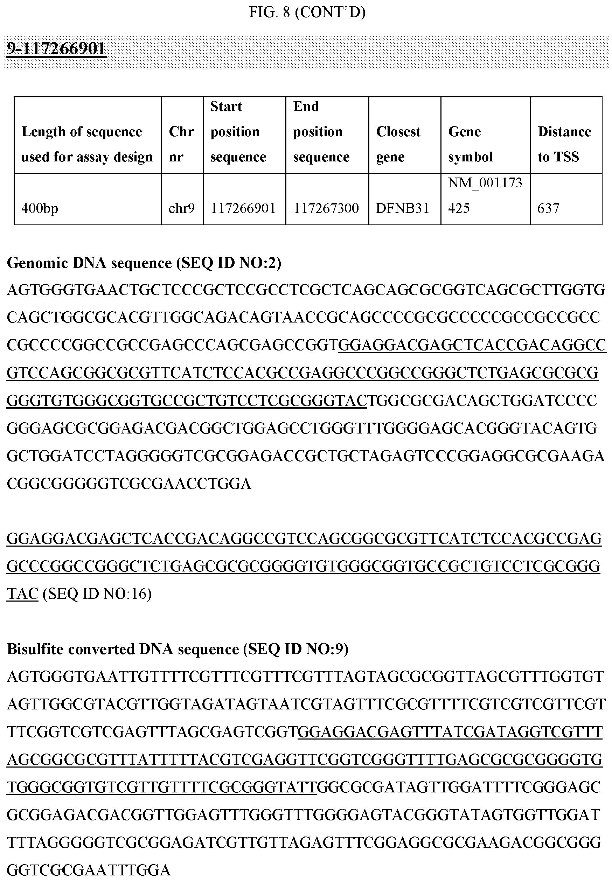

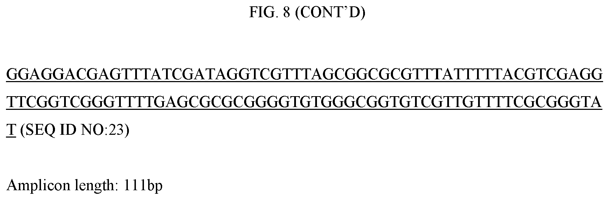

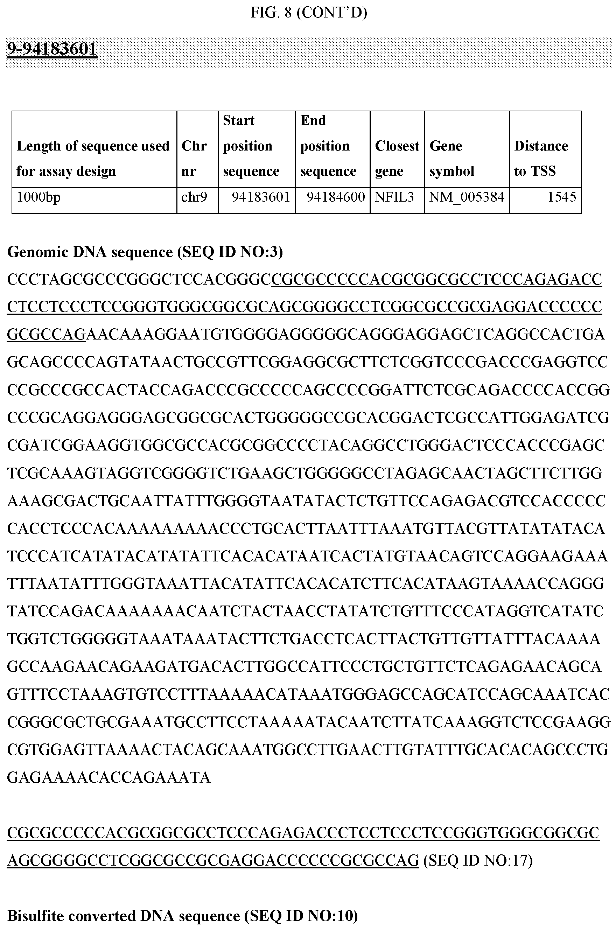

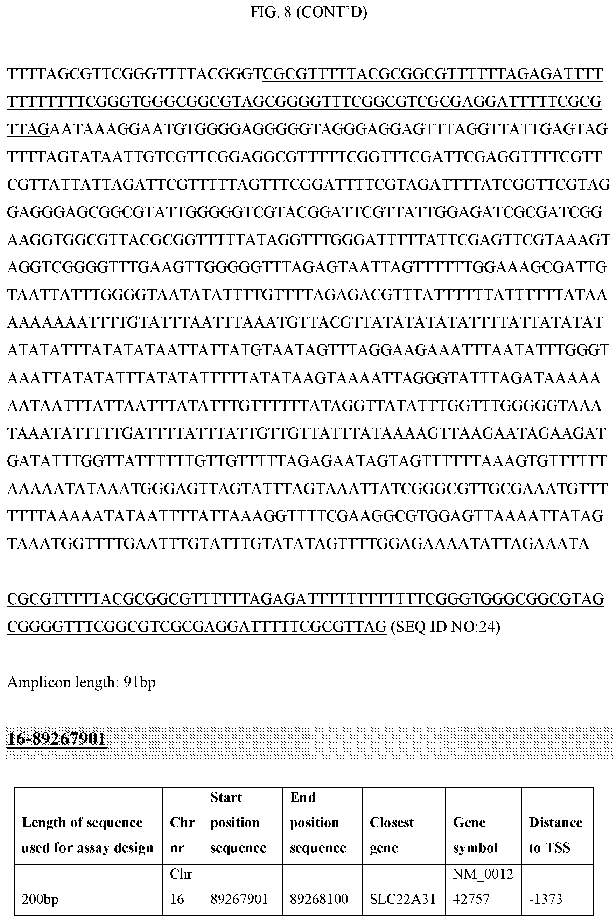

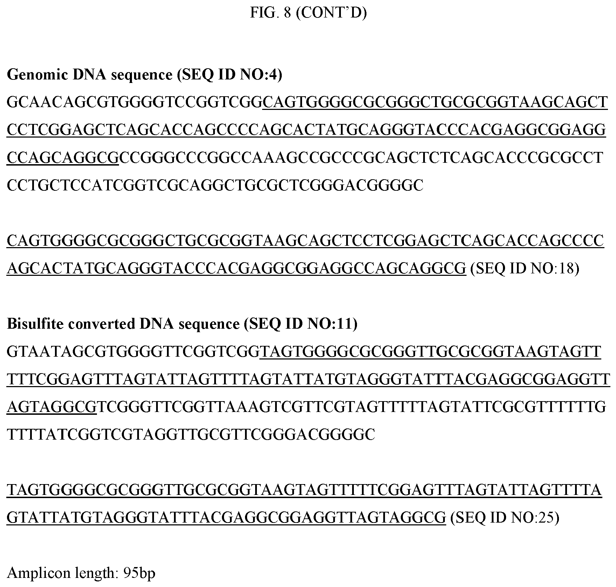

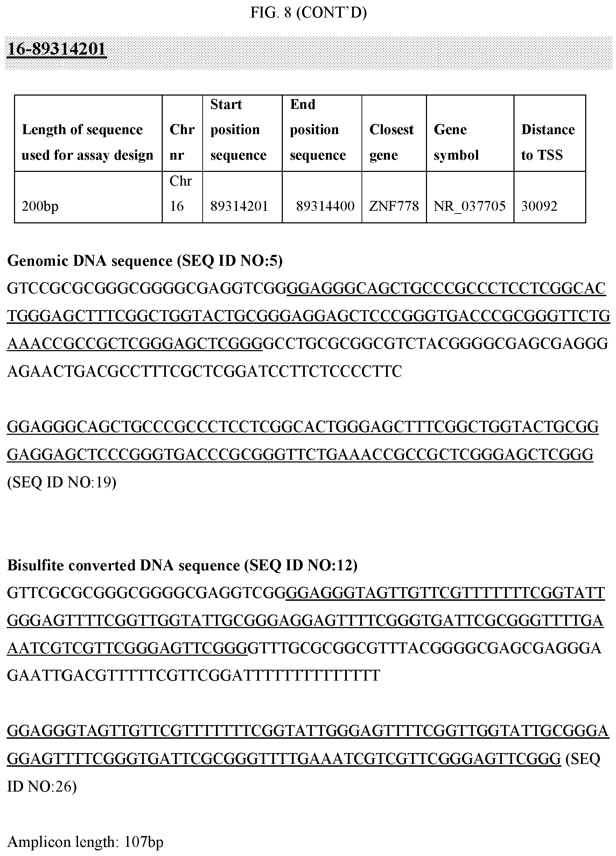

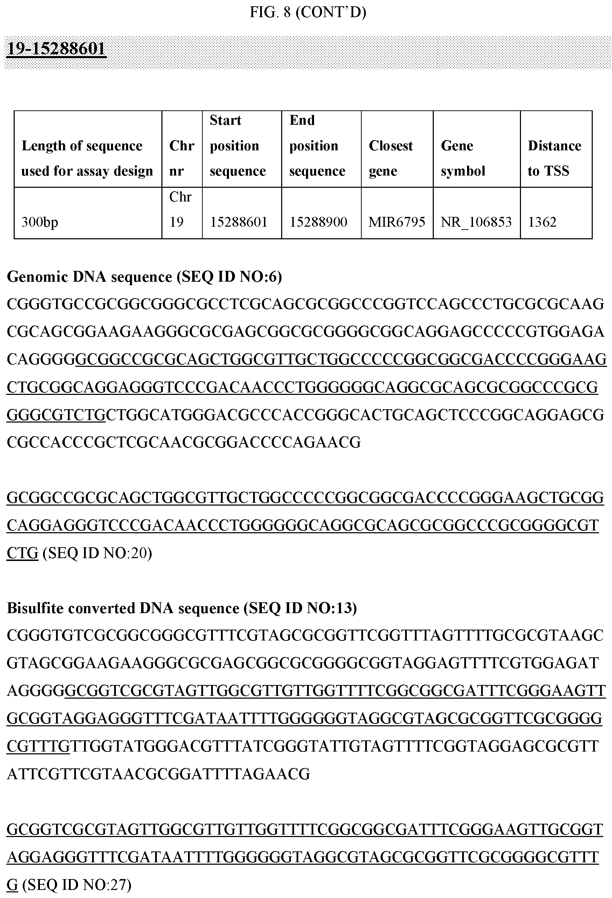

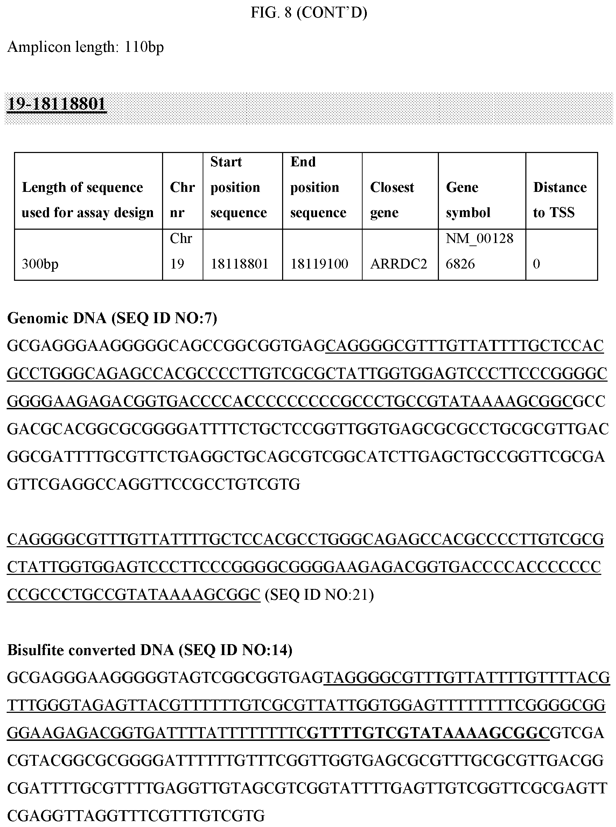

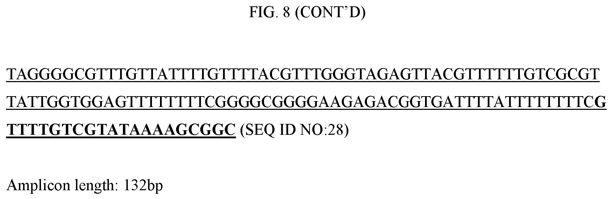

FIG. 8: Marker sequences and amplicons.
FIG. 9: Plot representing the clinical course for Patient 1.
FIG. 10: Plot representing the clinical course for Patient 2.
FIG. 11: Plot representing the clinical course for Patient 3.
FIG. 12: Plot representing the clinical course for Patient 4.
FIG. 13: Plot representing the clinical course for Patient 5.

### DEFINITIONS

To facilitate an understanding of the present invention, a number of terms and phrases are defined below:
As used herein, the term "sensitivity" is defined as a statistical measure of performance of an assay (e.g., method, test), calculated by dividing the number of true positives by the sum of the true positives and the false negatives.

As used herein, the term "specificity" is defined as a statistical measure of performance of an assay (e.g., method, test), calculated by dividing the number of true negatives by the sum of true negatives and false positives.

As used herein, the term "CpG island" refers to a genomic DNA region that contains a high percentage of CpG sites relative to the average genomic CpG incidence (per same species, per same individual, or per subpopulation (e.g., strain, ethnic subpopulation, or the like). Various parameters and definitions for CpG islands exist; for example, in some embodiments, CpG islands are defined as having a GC percentage that is greater than 50% and with an observed/expected CpG ratio that is greater than 60% (Gardiner-Garden et al. (1987) J Mol. Biol. 196:261-282; Baylin et al. (2006) Nat. Rev. Cancer 6:107-116; Irizarry et al. (2009) Nat. Genetics 41:178. 186) In some embodiments, CpG islands may have a GC content >55% and observed CpG/expected CpG of 0.65 (Takai et al. (2007) PNAS 99:3740-3745). Various parameters also exist regarding the length of CpG islands. As used herein, CpG islands may be less than 100 bp; 100-200 bp, 200-300 bp, 300-500 bp, 500-750 bp; 750-1000 bp; 1000 or more bp in length. In some embodiments, CpG islands show altered methylation patterns relative to controls.

As used herein, the term "CpG shore" or "CpG island shore" refers to a genomic region external to a CpG island that is or that has potential to have altered methylation (e.g., 5hmC) patterns (see, e.g., Irizarry et al. (2009) Nat. Genetics 41:178-186). CpG island shores may show altered methylation patterns relative to controls. CpG island shores may be located in various regions relative to CpG islands (see, e.g., Irizarry et al. (2009) Nat. Genetics 41;178-186). Accordingly, in some embodiments, CpG island shores are located less than 100 bp; 100-250 bp; 250-500 bp; 500-1000 bp; 1000-1500 bp; 1500-2000 bp; 2000-3000 bp; 3000 bp or more away from a CpG island.

As used herein, the "aberrant methylation" refers to a nucleic acid that has an altered level of methylation or methylation pattern. In some embodiments, the aberrant methylation is altered levels or patterns of methylation relative to a control nucleic acid (e.g., the corresponding nucleic acid from a patient not diagnosed with bladder cancer or an unmethylated nucleic acid control).

As used herein, the term "metastasis" is meant to refer to the process in which cancer cells originating in one organ or part of the body relocate to another part of the body and continue to replicate. Metastasized cells subsequently form tumors which may further metastasize. Metastasis thus refers to the spread of cancer from the part of the body where it originally occurs to other parts of the body.

As used herein, "an individual is suspected of being susceptible to metastasized cancer" is meant to refer to an individual who is at an above-average risk of developing metastasized cancer. Examples of individuals at a particular risk of developing cancer of a particular type (e.g., bladder cancer) are those whose family medical history indicates above average incidence of such cancer type among family members and/or those who have already developed cancer and have been effectively treated who therefore face a risk of relapse and recurrence. Other factors which may contribute to an above-average risk of developing metastasized cancer which would thereby lead to the classification of an individual as being suspected of being susceptible to metastasized cancer may be based upon an individual's specific genetic, medical and/or behavioral background and characteristics.

The term "neoplasm" as used herein refers to any new and abnormal growth of tissue. Thus, a neoplasm can be a premalignant neoplasm or a malignant neoplasm. The term "neoplasm-specific marker" refers to any biological material that can be used to indicate the presence of a neoplasm. Examples of biological materials include, without limitation, nucleic acids, polypeptides, carbohydrates, fatty acids, cellular components (e.g., cell membranes and mitochondria), and whole cells.

As used herein, the term "amplicon" refers to a nucleic acid generated using primer pairs. The amplicon is typically single-stranded DNA (e.g., the result of asymmetric amplification), however, it may be RNA or dsDNA.

The term "amplifying" or "amplification" in the context of nucleic acids refers to the production of multiple copies of a polynucleotide, or a portion of the polynucleotide, typically starting from a small amount of the polynucleotide (e.g., a single polynucleotide molecule), where the amplification products or amplicons are generally detectable. Amplification of polynucleotides encompasses a variety of chemical and enzymatic processes. The generation of multiple DNA copies from one or a few copies of a target or template DNA molecule during a polymerase chain reaction (PCR) or a ligase chain reaction (LCR; see, e.g., U.S. Patent No. 5,494,810) are forms of amplification. Additional types of amplification include, but are not limited to, allele-specific PCR (see, e.g., U.S. Patent No. 5,639,611), assembly PCR (see, e.g., U.S. Patent No. 5,965,408), helicase-dependent amplification (see, e.g., U.S. Patent No. 7,662,594), hot-start PCR (see, e.g., U.S. Patent Nos. 5,773,258 and 5,338,671), intersequence-specfic PCR, inverse PCR (see, e.g., Triglia, et al. (1988) Nucleic Acids Res., 16:8186), ligation-mediated PCR (see, e.g., Guilfoyle, R. et al., Nucleic Acids Research, 25:1854-1858 (1997); U.S. Patent No. 5,508,169), methylation-specific PCR (see, e.g., Herman, et al., (1996) PNAS 93(13) 9821-9826), miniprimer PCR, multiplex ligation-dependent probe amplification (see, e.g., Schouten, et al., (2002) Nucleic Acids Research 30(12): e57), multiplex PCR (see, e.g., Chamberlain, et al., (1988) Nucleic Acids Research 16(23) 11141-11156; Ballabio, et al., (1990) Human Genetics 84(6) 571-573; Hayden, et al., (2008) BMC Genetics 9:80), nested PCR, overlap-extension PCR (see, e.g., Higuchi, et al., (1988) Nucleic Acids Research 16(15) 7351-7367), real time PCR (see, e.g., Higuchi, etl al., (1992) Biotechnology 10:413-417; Higuchi, et al., (1993) Biotechnology 11:1026-1030), reverse transcription PCR (see, e.g., Bustin, S.A. (2000) J. Molecular Endocrinology 25:169-193), solid phase PCR, thermal asymmetric interlaced PCR, and Touchdown PCR (see, e.g., Don, et al., Nucleic Acids Research (1991) 19(14) 4008; Roux, K. (1994) Biotechniques 16(5) 812-814; Hecker, et al., (1996) Biotechniques 20(3) 478-485). Polynucleotide amplification also can be accomplished using digital PCR (see, e.g., Kalinina, et al., Nucleic Acids Research. 25; 1999-2004, (1997); Vogelstein and Kinzler, Proc Natl Acad Sci USA. 96; 9236-41, (1999); International Patent Publication No. WO05023091A2; US Patent Application Publication No. 20070202525).

As used herein, the terms "complementary" or "complementarity" are used in reference to polynucleotides (*i.e.,* a sequence of nucleotides) related by the base-pairing rules. For example, the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

As used herein, the term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced (*e.g.,* in the presence of nucleotides and an inducing agent such as a biocatalyst (*e.g*., a DNA polymerase or the like) and at a suitable temperature and pH). The primer is typically single stranded for maximum efficiency in amplification, but may alternatively be double stranded. If double stranded, the primer is generally first treated to separate its strands before being used to prepare extension products. In some embodiments, the primer is an oligodeoxyribonucleotide. The primer is sufficiently long to prime the synthesis of extension products in the presence of the inducing agent. The exact lengths of the primers will depend on many factors, including temperature, source of primer and the use of the method. In certain embodiments, the primer is a capture primer.

As used herein, the term "nucleic acid molecule" refers to any nucleic acid containing molecule, including but not limited to, DNA or RNA. The term encompasses sequences that include any of the known base analogs of DNA and RNA including, but not limited to, 4 acetylcytosine, 8-hydroxy-N6-methyladenosine, aziridinylcytosine, pseudoisocytosine, 5-(carboxyhydroxyl-methyl) uracil, 5-fluorouracil, 5-bromouracil, 5-carboxymethylaminomethyl-2-thiouracil, 5-carboxymethyl-aminomethyluracil, dihydrouracil, inosine, N6-isopentenyladenine, 1-methyladenine, 1-methylpseudo-uracil, 1-methylguanine, 1-methylinosine, 2,2-dimethyl-guanine, 2-methyladenine, 2-methylguanine, 3-methyl-cytosine, 5-methylcytosine, 5-hydroxymethylcytosine, b-glucosyl-5-hydroxymethylcytosine, 5-formylcytosine, and 5-carboxycytosine, N6-methyladenine, 7-methylguanine, 5-methylaminomethyluracil, 5-methoxy-amino-methyl-2-thiouracil, beta-D-mannosylqueosine, 5'-methoxycarbonylmethyluracil, 5-methoxyuracil, 2-methylthio-N-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

As used herein, the term "nucleobase" is synonymous with other terms in use in the art including "nucleotide," "deoxynucleotide," "nucleotide residue," "deoxynucleotide residue," "nucleotide triphosphate (NTP)," or deoxynucleotide triphosphate (dNTP).

An "oligonucleotide" refers to a nucleic acid that includes at least two nucleic acid monomer units (e.g., nucleotides), typically more than three monomer units, and more typically greater than ten monomer units. The exact size of an oligonucleotide generally depends on various factors, including the ultimate function or use of the oligonucleotide. To further illustrate, oligonucleotides are typically less than 200 residues long (e.g., between 15 and 100), however, as used herein, the term is also intended to encompass longer polynucleotide chains. Oligonucleotides are often referred to by their length. For example a 24 residue oligonucleotide is referred to as a "24-mer". Typically, the nucleoside monomers are linked by phosphodiester bonds or analogs thereof, including phosphorothioate, phosphorodithioate, phosphoroselenoate, phosphorodiselenoate, phosphoroanilothioate, phosphoranilidate, phosphoramidate, and the like, including associated counterions, e.g., H⁺, NH₄⁺, Na⁺, and the like, if such counterions are present. Further, oligonucleotides are typically single-stranded. Oligonucleotides are optionally prepared by any suitable method, including, but not limited to, isolation of an existing or natural sequence, DNA replication or amplification, reverse transcription, cloning and restriction digestion of appropriate sequences, or direct chemical synthesis by a method such as the phosphotriester method of Narang et al. (1979) Meth Enzymol. 68: 90-99; the phosphodiester method of Brown et al. (1979) Meth Enzymol. 68: 109-151; the diethylphosphoramidite method of Beaucage et al. (1981) Tetrahedron Lett. 22: 1859-1862; the triester method of Matteucci et al. (1981) J Am Chem Soc. 103:3185-3191; automated synthesis methods; or the solid support method of U.S. Pat. No. 4,458,066, entitled "PROCESS FOR PREPARING POLYNUCLEOTIDES," issued Jul. 3, 1984 to Caruthers et al., or other methods known to those skilled in the art. All of these references are incorporated by reference.

A "sequence" of a biopolymer refers to the order and identity of monomer units (e.g., nucleotides, etc.) in the biopolymer. The sequence (e.g., base sequence) of a nucleic acid is typically read in the 5' to 3' direction.

As used herein, the term "subject" refers to any animal (*e.g.,* a mammal), including, but not limited to, humans, non-human primates, rodents, and the like, which is to be the recipient of a particular treatment. Typically, the terms "subject" and "patient" are used interchangeably herein in reference to a human subject.

The term "gene" refers to a nucleic acid (*e.g.,* DNA) sequence that comprises coding sequences necessary for the production of a polypeptide, RNA (*e.g*., including but not limited to, mRNA, tRNA and rRNA) or precursor. The polypeptide, RNA, or precursor can be encoded by a full length coding sequence or by any portion of the coding sequence so long as the desired activity or functional properties (*e.g*., enzymatic activity, ligand binding, signal transduction, etc.) of the full-length or fragment are retained. The term also encompasses the coding region of a structural gene and the including sequences located adjacent to the coding region on both the 5' and 3' ends for a distance of about 1 kb on either end such that the gene corresponds to the length of the full-length mRNA. The sequences that are located 5' of the coding region and which are present on the mRNA are referred to as 5' untranslated sequences. The sequences that are located 3' or downstream of the coding region and that are present on the mRNA are referred to as 3' untranslated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) processed transcript. The mRNA functions during translation to specify the sequence or order of amino acids in a nascent polypeptide.

### DETAILED DESCRIPTION OF THE INVENTION

A non-invasive, cost-efficient and accurate test for detection of bladder cancer would largely facilitate the clinical management of patients and reduce the economic burden related to this malignancy. However, robust and reliable biomarkers are still lacking, and despite several FDA-approved urine-based tests and a variety of biomarkers proposed in the literature, cystoscopy remains the gold standard. Here, we present a panel of urine-based DNA methylation biomarkers for highly sensitive and specific detection of bladder cancer. Given its high performance, the non-invasive and inexpensive nature, as well as the simple detection workflow, the panel improves both early detection and monitoring for recurrence.

Based on a patient survey, the sensitivity of a molecular urinary marker should attain at least 90%, and preferably 95%, in order to be considered acceptable as a replacement for cystoscopy³⁸. Moreover, high specificity is essential in order to avoid unnecessary anxiety and treatment costs. In a recent systematic review of methylation markers in urine for the detection of bladder cancer, only eight out of 43 studies reported marker panels with both high sensitivity (>90%) and specificity (>80%)¹⁵. In comparison, the urine-based methylation panel identified in the present study detects bladder cancer with 96% sensitivity and 100% specificity in a discovery series. Application of the biomarkers in monitoring of patients for recurrence following TURB also indicates a diagnostic performance that supports clinical utility for (partial) replacement of cystoscopy in the monitoring setting. Finally, the test is based on ddPCR-technology, which is relatively quick, simple and inexpensive compared to other methods, such as microarrays and high throughput sequencing^{39,40}.

Several of the methylation markers identified in the present study display co-variance. Only three markers were sufficient to correctly identify 25 out of 26 bladder cancer cases in our discovery cohort. Furthermore, different combinations of markers may be optimal for different clinical utilities. In a monitoring setting, the use of patient-specific marker combinations may be advantageous, because the marker concentrations would in this case be followed over time, and each patient would serve as is its own control.

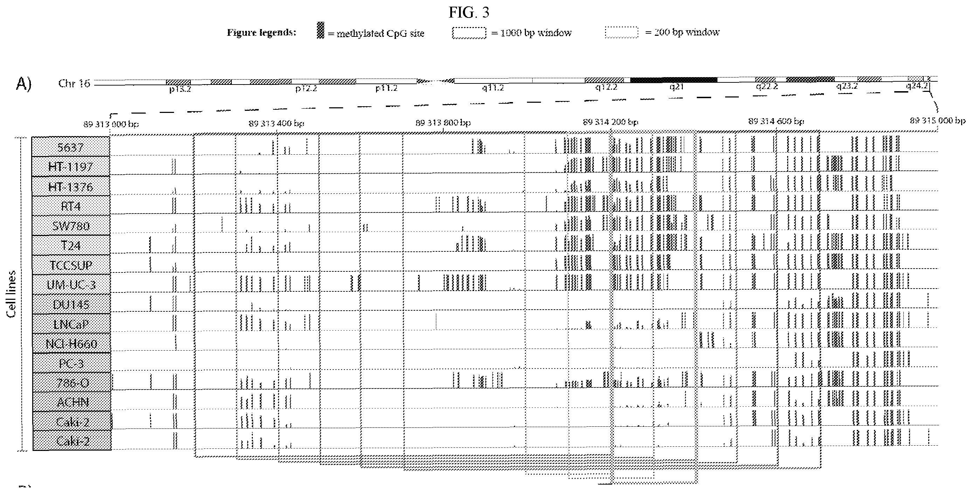

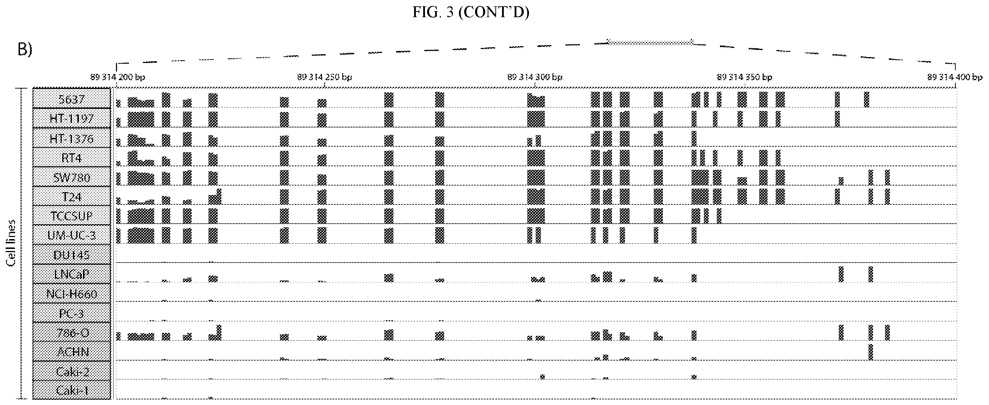

The methylation markers in the present study represent a 'novel generation' of biomarkers identified using a methylome sequencing approach and an in-house established bioinformatic pipeline. The majority of DNA methylation biomarker research has focused on defined regions of the genome with known biological function, such as promoters⁴¹. In contrast, the use of methylome sequencing allows exploring parts of the genome that have previously been inaccessible with targeted approaches. Interestingly, the majority (69%) of the markers identified in the present study are located outside of promoter regions, *i.e.* more than 1500bp from the nearest transcription start site (Figure 3).

Accordingly, provided herein are compositions and methods for detecting aberrant methylation in one or more (e.g., 1, 2, 3, 4, 5, 6, or 7) markers described by SEQ ID NOs: 1-7 or 15-21 and uses thereof. The present disclosure is not limited to particular methods of detecting the methylation status of the markers. In some embodiments, additional markers are detected (e.g., VIM). Exemplary methods are described herein.

Methylation status of the markers described herein can be detected in any sample, prior to or after additional processing steps. Examples of suitable sample types include, but are not limited to, urine, blood, and tissue (e.g., bladder) samples.

In some embodiments, the methylation levels or patterns are compared to the levels or patterns in control nucleic acids (e.g., corresponding nucleic acids from subjects not diagnosed with bladder cancer).

The most frequently used method for analyzing a nucleic acid for the presence of 5-methylcytosine is based upon the bisulfite method described by Frommer, et al. for the detection of 5-methylcytosines in DNA (Frommer et al. (1992) Proc. Natl. Acad. Sci. USA 89: 1827-31) or variations thereof. The bisulfite method of mapping 5-methylcytosines is based on the observation that cytosine, but not 5-methylcytosine, reacts with hydrogen sulfite ion (also known as bisulfite). The reaction is usually performed according to the following steps: first, cytosine reacts with hydrogen sulfite to form a sulfonated cytosine. Next, spontaneous deamination of the sulfonated reaction intermediate results in a sulfonated uracil. Finally, the sulfonated uricil is desulfonated under alkaline conditions to form uracil. Detection is possible because uracil forms base pairs with adenine (thus behaving like thymine), whereas 5-methylcytosine base pairs with guanine (thus behaving like cytosine). This makes the discrimination of methylated cytosines from non-methylated cytosines possible by, e.g., bisulfite genomic sequencing (Grigg G, & Clark S, Bioessays (1994) 16: 431-36; Grigg G, DNA Seq. (1996) 6: 189-98) or methylation-specific PCR (MSP) as is disclosed, e.g., in U.S. Patent No. 5,786,146.

Some conventional technologies are related to methods comprising enclosing the DNA to be analyzed in an agarose matrix, thereby preventing the diffusion and renaturation of the DNA (bisulfite only reacts with single-stranded DNA), and replacing precipitation and purification steps with a fast dialysis (Olek A, et al. (1996) "A modified and improved method for bisulfite based cytosine methylation analysis" Nucleic Acids Res. 24: 5064-6). It is thus possible to analyze individual cells for methylation status, illustrating the utility and sensitivity of the method. An overview of conventional methods for detecting 5-methylcytosine is provided by Rein, T., et al. (1998) Nucleic Acids Res. 26: 2255.

The bisulfite technique typically involves amplifying short, specific fragments of a known nucleic acid subsequent to a bisulfite treatment, then either assaying the product by sequencing (Olek & Walter (1997) Nat. Genet. 17: 275-6) or a primer extension reaction (Gonzalgo & Jones (1997) Nucleic Acids Res. 25: 2529-31; WO 95/00669; U.S. Pat. No. 6,251,594) to analyze individual cytosine positions. Some methods use enzymatic digestion (Xiong & Laird (1997) Nucleic Acids Res. 25: 2532-4). Detection by hybridization has also been described in the art (Olek et al., WO 99/28498). Additionally, use of the bisulfite technique for methylation detection with respect to individual genes has been described (Grigg & Clark (1994) Bioessays 16: 431-6,; Zeschnigk et al. (1997) Hum Mol Genet. 6: 387-95; Feil et al. (1994) Nucleic Acids Res. 22: 695; Martin et al. (1995) Gene 157: 261-4; WO 9746705; WO 9515373).

Various methylation assay procedures are known in the art and can be used in conjunction with bisulfite treatment according to the present technology. These assays allow for determination of the methylation state of one or a plurality of CpG dinucleotides (e.g., CpG islands) within a nucleic acid sequence. Such assays involve, among other techniques, sequencing of bisulfite-treated nucleic acid, PCR (for sequence-specific amplification), Southern blot analysis, and use of methylation-sensitive restriction enzymes.

For example, genomic sequencing has been simplified for analysis of methylation patterns and 5-methylcytosine distributions by using bisulfite treatment (Frommer et al. (1992) Proc. Natl. Acad. Sci. USA 89: 1827-1831). Additionally, restriction enzyme digestion of PCR products amplified from bisulfite-converted DNA finds use in assessing methylation state, e.g., as described by Sadri & Hornsby (1997) Nucl. Acids Res. 24: 5058-5059 or as embodied in the method known as COBRA (Combined Bisulfite Restriction Analysis) (Xiong & Laird (1997) Nucleic Acids Res. 25: 2532-2534).

COBRA^{™} analysis is a quantitative methylation assay useful for determining DNA methylation levels at specific loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (e.g., as might be found in a typical COBRA^{™}-based kit) for COBRA^{™} analysis may include, but are not limited to: PCR primers for specific loci (e.g., specific genes, markers, DMR, regions of genes, regions of markers, bisulfite treated DNA sequence , CpG island, etc.); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components.

Preferably, assays such as "MethyLight^{™}" (a fluorescence-based real-time PCR technique) (Eads et al., Cancer Res. 59:2302-2306, 1999), Ms-SNuPE^{™} (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; U.S. Pat. No. 5,786,146), and methylated CpG island amplification ("MCA"; Toyota et al., Cancer Res. 59:2307-12, 1999) are used alone or in combination with one or more of these methods.

The "HeavyMethyl^{™}" assay, technique is a quantitative method for assessing methylation differences based on methylation-specific amplification of bisulfite-treated DNA. Methylation-specific blocking probes ("blockers") covering CpG positions between, or covered by, the amplification primers enable methylation-specific selective amplification of a nucleic acid sample.

The term "HeavyMethyl^{™} MethyLight^{™}" assay refers to a HeavyMethyl^{™} MethyLight^{™} assay, which is a variation of the MethyLight^{™} assay, wherein the MethyLight^{™} assay is combined with methylation specific blocking probes covering CpG positions between the amplification primers. The HeavyMethyl^{™} assay may also be used in combination with methylation specific amplification primers.

Typical reagents (e.g., as might be found in a typical MethyLight^{™}-based kit) for HeavyMethyl^{™} analysis may include, but are not limited to: PCR primers for specific loci (e.g., specific genes, markers, DMR, regions of genes, regions of markers, bisulfite treated DNA sequence , CpG island, or bisulfite treated DNA sequence or CpG island, etc.); blocking oligonucleotides; optimized PCR buffers and deoxynucleotides; and Taq polymerase.

MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; U.S. Pat. No. 5,786,146). Briefly, DNA is modified by sodium bisulfite, which converts unmethylated, but not methylated cytosines, to uracil, and the products are subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (e.g., as might be found in a typical MSP-based kit) for MSP analysis may include, but are not limited to: methylated and unmethylated PCR primers for specific loci (e.g., specific genes, markers, DMR, regions of genes, regions of markers, bisulfite treated DNA sequence, CpG island, etc.); optimized PCR buffers and deoxynucleotides, and specific probes.

The MethyLight^{™} assay is a high-throughput quantitative methylation assay that utilizes fluorescence-based real-time PCR (e.g., TaqMan^{®}) that requires no further manipulations after the PCR step (Eads et al., Cancer Res. 59:2302-2306, 1999). Briefly, the MethyLight^{™} process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed in a "biased" reaction, e.g., with PCR primers that overlap known CpG dinucleotides. Sequence discrimination occurs both at the level of the amplification process and at the level of the fluorescence detection process.

The MethyLight^{™} assay is used as a quantitative test for methylation patterns in a nucleic acid, e.g., a genomic DNA sample, wherein sequence discrimination occurs at the level of probe hybridization. In a quantitative version, the PCR reaction provides for a methylation specific amplification in the presence of a fluorescent probe that overlaps a particular putative methylation site. An unbiased control for the amount of input DNA is provided by a reaction in which neither the primers, nor the probe, overlie any CpG dinucleotides. Alternatively, a qualitative test for genomic methylation is achieved by probing the biased PCR pool with either control oligonucleotides that do not cover known methylation sites (e.g., a fluorescence-based version of the HeavyMethyl^{™} and MSP techniques) or with oligonucleotides covering potential methylation sites.

The MethyLight^{™} process is used with any suitable probe (e.g. a "TaqMan^{®}" probe, a Lightcycler^{®} probe, etc.) For example, in some applications double-stranded genomic DNA is treated with sodium bisulfite and subjected to one of two sets of PCR reactions using TaqMan^{®} probes, e.g., with MSP primers and/or HeavyMethyl blocker oligonucleotides and a TaqMan^{®} probe. The TaqMan^{®} probe is dual-labeled with fluorescent "reporter" and "quencher" molecules and is designed to be specific for a relatively high GC content region so that it melts at about a 10°C higher temperature in the PCR cycle than the forward or reverse primers. This allows the TaqMan^{®} probe to remain fully hybridized during the PCR annealing/extension step. As the Taq polymerase enzymatically synthesizes a new strand during PCR, it will eventually reach the annealed TaqMan^{®} probe. The Taq polymerase 5' to 3' endonuclease activity will then displace the TaqMan^{®} probe by digesting it to release the fluorescent reporter molecule for quantitative detection of its now unquenched signal using a real-time fluorescent detection system.

Typical reagents (e.g., as might be found in a typical MethyLight^{™}-based kit) for MethyLight^{™} analysis may include, but are not limited to: PCR primers for specific loci (e.g., specific genes, markers, DMR, regions of genes, regions of markers, bisulfite treated DNA sequence, CpG island, etc.); TaqMan^{®} or Lightcycler^{®} probes; optimized PCR buffers and deoxynucleotides; and Taq polymerase.

The QM^{™} (quantitative methylation) assay is an alternative quantitative test for methylation patterns in genomic DNA samples, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe, overlie any CpG dinucleotides. Alternatively, a qualitative test for genomic methylation is achieved by probing the biased PCR pool with either control oligonucleotides that do not cover known methylation sites (a fluorescence-based version of the HeavyMethyl^{™} and MSP techniques) or with oligonucleotides covering potential methylation sites.

The QM^{™} process can be used with any suitable probe, e.g., "TaqMan^{®}" probes, Lightcycler^{®} probes, in the amplification process. For example, double-stranded genomic DNA is treated with sodium bisulfite and subjected to unbiased primers and the TaqMan^{®} probe. The TaqMan^{®} probe is dual-labeled with fluorescent "reporter" and "quencher" molecules, and is designed to be specific for a relatively high GC content region so that it melts out at about a 10°C higher temperature in the PCR cycle than the forward or reverse primers. This allows the TaqMan^{®} probe to remain fully hybridized during the PCR annealing/extension step. As the Taq polymerase enzymatically synthesizes a new strand during PCR, it will eventually reach the annealed TaqMan^{®} probe. The Taq polymerase 5' to 3' endonuclease activity will then displace the TaqMan^{®} probe by digesting it to release the fluorescent reporter molecule for quantitative detection of its now unquenched signal using a real-time fluorescent detection system. Typical reagents (e.g., as might be found in a typical QM^{™}-based kit) for QM^{™} analysis may include, but are not limited to: PCR primers for specific loci (e.g., specific genes, markers, DMR, regions of genes, regions of markers, bisulfite treated DNA sequence, CpG island, etc.); TaqMan^{®} or Lightcycler^{®} probes; optimized PCR buffers and deoxynucleotides; and Taq polymerase.

The Ms-SNuPE^{™} technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site of interest. Small amounts of DNA can be analyzed (e.g., microdissected pathology sections) and it avoids utilization of restriction enzymes for determining the methylation status at CpG sites.

Typical reagents (e.g., as might be found in a typical Ms-SNuPE^{™}-based kit) for Ms-SNuPE^{™} analysis may include, but are not limited to: PCR primers for specific loci (e.g., regions of markers, bisulfite treated DNA sequence, CpG island, etc.); optimized PCR buffers and deoxynucleotides; gel extraction kit; positive control primers; Ms-SNuPE^{™} primers for specific loci; reaction buffer (for the Ms-SNuPE reaction); and labeled nucleotides. Additionally, bisulfite conversion reagents may include: DNA denaturation buffer; sulfonation buffer; DNA recovery reagents or kit (e.g., precipitation, ultrafiltration, affinity column); desulfonation buffer; and DNA recovery components.

Reduced Representation Bisulfite Sequencing (RRBS) begins with bisulfite treatment of nucleic acid to convert all unmethylated cytosines to uracil, followed by restriction enzyme digestion (e.g., by an enzyme that recognizes a site including a CG sequence such as MspI) and complete sequencing of fragments after coupling to an adapter ligand. The choice of restriction enzyme enriches the fragments for CpG dense regions, reducing the number of redundant sequences that may map to multiple gene positions during analysis. As such, RRBS reduces the complexity of the nucleic acid sample by selecting a subset (e.g., by size selection using preparative gel electrophoresis) of restriction fragments for sequencing. As opposed to whole-genome bisulfite sequencing, every fragment produced by the restriction enzyme digestion contains DNA methylation information for at least one CpG dinucleotide. As such, RRBS enriches the sample for promoters, CpG islands, and other genomic features with a high frequency of restriction enzyme cut sites in these regions and thus provides an assay to assess the methylation state of one or more genomic loci.

A typical protocol for RRBS comprises the steps of digesting a nucleic acid sample with a restriction enzyme such as MspI, filling in overhangs and A-tailing, ligating adaptors, bisulfite conversion, and PCR. See, e.g., et al. (2005) "Genome-scale DNA methylation mapping of clinical samples at single-nucleotide resolution" Nat Methods 7: 133-6; Meissner et al. (2005) "Reduced representation bisulfite sequencing for comparative high-resolution DNA methylation analysis" Nucleic Acids Res. 33: 5868-77.

In some embodiments, a quantitative allele-specific real-time target and signal amplification (QuARTS) assay is used to evaluate methylation state. Three reactions sequentially occur in each QuARTS assay, including amplification (reaction 1) and target probe cleavage (reaction 2) in the primary reaction; and FRET cleavage and fluorescent signal generation (reaction 3) in the secondary reaction. When target nucleic acid is amplified with specific primers, a specific detection probe with a flap sequence loosely binds to the amplicon. The presence of the specific invasive oligonucleotide at the target binding site causes cleavase to release the flap sequence by cutting between the detection probe and the flap sequence. The flap sequence is complementary to a nonhairpin portion of a corresponding FRET cassette. Accordingly, the flap sequence functions as an invasive oligonucleotide on the FRET cassette and effects a cleavage between the FRET cassette fluorophore and a quencher, which produces a fluorescent signal. The cleavage reaction can cut multiple probes per target and thus release multiple fluorophore per flap, providing exponential signal amplification. QuARTS can detect multiple targets in a single reaction well by using FRET cassettes with different dyes. See, e.g., in Zou et al. (2010) "Sensitive quantification of methylated markers with a novel methylation specific technology" Clin Chem 56: A199; U.S. Pat. Appl. Ser. Nos. 12/946,737, 12/946,745, 12/946,752, and 61/548,639.

The term "bisulfite reagent" refers to a reagent comprising bisulfite, disulfite, hydrogen sulfite, or combinations thereof, useful as disclosed herein to distinguish between methylated and unmethylated CpG dinucleotide sequences. Methods of said treatment are known in the art (e.g., PCT/EP2004/011715). It is preferred that the bisulfite treatment is conducted in the presence of denaturing solvents such as but not limited to n-alkylenglycol or diethylene glycol dimethyl ether (DME), or in the presence of dioxane or dioxane derivatives. In some embodiments the denaturing solvents are used in concentrations between 1% and 35% (v/v). In some embodiments, the bisulfite reaction is carried out in the presence of scavengers such as but not limited to chromane derivatives, e.g., 6-hydroxy-2,5,7,8,-tetramethylchromane 2-carboxylic acid or trihydroxybenzone acid and derivates thereof, e.g., Gallic acid (see: PCT/EP2004/011715). The bisulfite conversion is preferably carried out at a reaction temperature between 30°C and 70°C, whereby the temperature is increased to over 85°C for short times during the reaction (see: PCT/EP2004/011715). The bisulfite treated DNA is preferably purified prior to the quantification. This may be conducted by any means known in the art, such as but not limited to ultrafiltration, e.g., by means of Microcon^{™} columns (manufactured by Millipore^{™}). The purification is carried out according to a modified manufacturer's protocol (see, e.g., PCT/EP2004/011715).

The fragments obtained by means of the amplification can carry a directly or indirectly detectable label. In some embodiments, the labels are fluorescent labels, radionuclides, or detachable molecule fragments having a typical mass that can be detected in a mass spectrometer. Where said labels are mass labels, some embodiments provide that the labeled amplicons have a single positive or negative net charge, allowing for better delectability in the mass spectrometer. The detection may be carried out and visualized by means of, e.g., matrix assisted laser desorption/ionization mass spectrometry (MALDI) or using electron spray mass spectrometry (ESI).

Additional methods for detecting methylated nucleic acid are described below.

Mass spectrometry is a very sensitive and reliable analytical method to detect DNA methylation. MS in general is however not informative about the sequence context of the methylation, thus limited in studying the function of this DNA modification.

Whole genome bisulfite sequencing, also known as BS-Seq, which is a high-throughput genome-wide analysis of DNA methylation. It is based on aforementioned sodium bisulfite conversion of genomic DNA, which is then sequenced on a Next-generation sequencing platform. The sequences obtained are then re-aligned to the reference genome to determine methylation states of CpG dinucleotides based on mismatches resulting from the conversion of unmethylated cytosines into uracil.

Reduced representation bisulfite sequencing, also known as RRBS knows several working protocols. The first RRBS protocol was called RRBS and aims for around 10% of the methylome, a reference genome is needed. Later came more protocols that were able to sequence a smaller portion of the genome and higher sample multiplexing. EpiGBS was the first protocol were you could multiplex 96 sample in one lane of Illumina sequencing and were a reference genome was no longer needed. A de novo reference construction from the Watson and Crick reads made population screening of SNP's and SMP's simultaneously a fact.

The HELP assay, which is based on restriction enzymes' differential ability to recognize and cleave methylated and unmethylated CpG DNA sites.

GLAD-PCR assay, which is based on new type of enzymes - site-specific methyl-directed DNA endonucleases, which hydrolyze only methylated DNA.

ChIP-on-chip assays, which is based on the ability of commercially prepared antibodies to bind to DNA methylation-associated proteins like MeCP2.

Restriction landmark genomic scanning, a complicated and now rarely used assay based upon restriction enzymes' differential recognition of methylated and unmethylated CpG sites; the assay is similar in concept to the HELP assay.

Methylated DNA immunoprecipitation (MeDIP), analogous to chromatin immunoprecipitation, immunoprecipitation is used to isolate methylated DNA fragments for input into DNA detection methods such as DNA microarrays (MeDIP-chip) or DNA sequencing (MeDIP-seq).

Pyrosequencing of bisulfite treated DNA. This is sequencing of an amplicon made by a normal forward primer but a biotinylated reverse primer to PCR the gene of choice. The Pyrosequencer then analyses the sample by denaturing the DNA and adding one nucleotide at a time to the mix according to a sequence given by the user. If there is a mis-match, it is recorded and the percentage of DNA for which the mis-match is present is noted. This gives the user a percentage methylation per CpG island.

Molecular break light assay for DNA adenine methyltransferase activity - an assay that relies on the specificity of the restriction enzyme DpnI for fully methylated (adenine methylation) GATC sites in an oligonucleotide labeled with a fluorophore and quencher. The adenine methyltransferase methylates the oligonucleotide making it a substrate for DpnI. Cutting of the oligonucleotide by DpnI gives rise to a fluorescence increase.

Methyl Sensitive Southern Blotting is similar to the HELP assay, although uses Southern blotting techniques to probe gene-specific differences in methylation using restriction digests. This technique is used to evaluate local methylation near the binding site for the probe.

MethylCpG Binding Proteins (MBPs) and fusion proteins containing just the Methyl Binding Domain (MBD) are used to separate native DNA into methylated and unmethylated fractions. The percentage methylation of individual CpG islands can be determined by quantifying the amount of the target in each fraction. Extremely sensitive detection can be achieved in FFPE tissues with abscription-based detection.

High Resolution Melt Analysis (HRM or HRMA), is a post-PCR analytical technique. The target DNA is treated with sodium bisulfite, which chemically converts unmethylated cytosines into uracils, while methylated cytosines are preserved. PCR amplification is then carried out with primers designed to amplify both methylated and unmethylated templates. After this amplification, highly methylated DNA sequences contain a higher number of CpG sites compared to unmethylated templates, which results in a different melting temperature that can be used in quantitative methylation detection.

In some embodiments, digital droplet PCR is used to detect the markers described herein (See e.g., Hindson, B. J. et al. High-throughput droplet digital PCR system for absolute quantitation of DNA copy number. Anal Chem 83, 8604-8610, doi:10.1021/ac202028g (2011) and Hindson, C. M. et al. Absolute quantification by droplet digital PCR versus analog real-time PCR. Nat Methods 10, 1003-1005, doi:10.1038/nmeth.2633 (2013)).

Examples of commercially available digital droplet PCR systems include, but are not limited to, Droplet Digital PCR (ddPCR^{™}) System from Bio-Rad (Hercules, CA), RainStorm^{™} digital droplet technology from RainDance (Billerica, MA), and Crystal Digital PCR from Stilla (Villejuif, France).

The compositions and method described herein find use in the diagnosis and characterization of bladder cancer in a subject. In some embodiments, the methods described herein find use in diagnosis of bladder cancer in a subject not previously diagnosed with bladder cancer (e.g., a subject suspected of having bladder cancer based on symptoms or tests or a subject at risk of bladder cancer).

In some embodiments, the methods described herein find use in diagnosing recurrent (e.g., primary or metastatic) bladder cancer in a subject previously diagnosed with bladder cancer. For example, in some embodiments, the assay described herein is performed at regular intervals (e.g., monthly, once every several months, yearly, or another interval) in a subject previously treated for bladder cancer (e.g., post-surgery or other treatment).

In some embodiments, the results described herein are used to determine a treatment course or action or treat bladder cancer (e.g., recurrent or primary bladder cancer) in a subject. In some embodiments, subjects identified as having aberrant methylation in a marker described herein are administered a treatment for bladder cancer (e.g., surgery, radiation, chemotherapy, or immunotherapy).

Suitable surgical procedures that may be used in the methods as described herein include but are not limited one or more of transurethral resection, radial cystectomy, partial cystectomy, and urinary diversion.

Suitable radiation procedures that may be used in the methods as described hereininclude but are not limited to external radiation therapies and internal radiation therapies.

Suitable chemotherapies and immunotherapies that may be used in the methods as described herein include nut are not limited to administration of Tecentriq (Atezolizumab), Avelumab, Balversa (Erdafitinib), Bavencio (Avelumab), Cisplatin, Doxorubicin Hydrochloride, Durvalumab, Erdafitinib, Imfinzi (Durvalumab), Keytruda (Pembrolizumab), Opdivo (Nivolumab), Thiotepa and Valstar (Valrubicin). In some embodiments, the therapy admimnistered may be a combination therapy such as Gemcitabine/Cisplatin or MVAC (Methotrexate, Vinblastine Sulfate, Doxorubicin Hydrochloride, and Cisplatin).

A medical professional can communicate the assay results to a patient or a patient's family. In some cases, a medical professional can provide a patient and/or a patient's family with information regarding neoplasia, including treatment options, prognosis, and referrals to specialists, e.g., oncologists and/or radiologists. In some cases, a medical professional can provide a copy of a patient's medical records to communicate assay results to a specialist. A research professional can apply information regarding a subject's assay results to advance neoplasm research. For example, a researcher can compile data on the assay results, with information regarding the efficacy of a drug for treatment of neoplasia to identify an effective treatment. In some cases, a research professional can obtain assay results to evaluate a subject's enrollment, or continued participation in a research study or clinical trial. In some cases, a research professional can classify the severity of a subject's condition, based on assay results. In some cases, a research professional can communicate a subject's assay results to a medical professional. In some cases, a research professional can refer a subject to a medical professional for clinical assessment of neoplasia, and treatment thereof. Any appropriate method can be used to communicate information to another person (e.g., a professional). For example, information can be given directly or indirectly to a professional. For example, a laboratory technician can input the assay results into a computer-based record. In some cases, information is communicated by making a physical alteration to medical or research records. For example, a medical professional can make a permanent notation or flag a medical record for communicating a diagnosis to other medical professionals reviewing the record. In addition, any type of communication can be used to communicate the information. For example, mail, e-mail, telephone, and face-to-face interactions can be used. The information also can be communicated to a professional by making that information electronically available to the professional. For example, the information can be communicated to a professional by placing the information on a computer database such that the professional can access the information. In addition, the information can be communicated to a hospital, clinic, or research facility serving as an agent for the professional.

The present disclosure also provides a variety of computer-related embodiments. Specifically, in some embodiments the disclosure provides computer programming for analyzing and comparing a pattern of marker methylation detection results in a sample obtained from a subject to, for example, a library of such marker patterns known to be indicative of the presence or absence of bladder cancer.

In some embodiments, the present disclosure provides computer programming for analyzing and comparing a first and a second pattern of marker methylation detection results from a sample taken at least two different time points. In some embodiments, the first pattern may be indicative of a pre-cancerous condition and/or low risk condition for cancer and/or progression from a pre-cancerous condition to a cancerous condition. In such embodiments, the comparing provides for monitoring of the progression of the condition from the first time point to the second time point.

In yet another embodiment, the disclosure provides computer programming for analyzing and comparing a pattern of marker methylation detection results from a sample to a library of bladder cancer-specific marker methylation patterns known to be indicative of the presence or absence of a cancer, wherein the comparing provides, for example, a differential diagnosis between a benign neoplasm, and an aggressively malignant neoplasm (e.g., the marker pattern provides for staging and/or grading of the cancerous condition).

The methods and systems described herein can be implemented in numerous ways. In one embodiment, the methods involve use of a communications infrastructure, for example the internet. Several embodiments of the disclosure are discussed below. It is also to be understood that the present disclosure may be implemented in various forms of hardware, software, firmware, processors, distributed servers (e.g., as used in cloud computing) or a combination thereof. The methods and systems described herein can be implemented as a combination of hardware and software. The software can be implemented as an application program tangibly embodied on a program storage device, or different portions of the software implemented in the user's computing environment (e.g., as an applet) and on the reviewer's computing environment, where the reviewer may be located at a remote site (e.g., at a service provider's facility).

For example, during or after data input by the user, portions of the data processing can be performed in the user-side computing environment. For example, the user-side computing environment can be programmed to provide for defined test codes to denote platform, carrier/diagnostic test, or both; processing of data using defined flags, and/or generation of flag configurations, where the responses are transmitted as processed or partially processed responses to the reviewer's computing environment in the form of test code and flag configurations for subsequent execution of one or more algorithms to provide a results and/or generate a report in the reviewer's computing environment.

The application program for executing the algorithms described herein may be uploaded to, and executed by, a machine comprising any suitable architecture. In general, the machine involves a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the application program (or a combination thereof) which is executed via the operating system. In addition, various other peripheral devices may be connected to the computer platform such as an additional data storage device and a printing device.

As a computer system, the system generally includes a processor unit. The processor unit operates to receive information, which generally includes test data (e.g., specific gene products assayed), and test result data (e.g., the pattern of neoplasm-specific marker (e.g., epigenetic marker, 5hmC modification) detection results from a sample). This information received can be stored at least temporarily in a database, and data analyzed in comparison to a library of marker patterns known to be indicative of the presence or absence of a pre-cancerous condition, or known to be indicative of a stage and/or grade of cancer.

Part or all of the input and output data can also be sent electronically; certain output data (e.g., reports) can be sent electronically or telephonically (e.g., by facsimile, e.g., using devices such as fax back). Exemplary output receiving devices can include a display element, a printer, a facsimile device and the like. Electronic forms of transmission and/or display can include email, interactive television, and the like. In some embodiments, all or a portion of the input data and/or all or a portion of the output data (e.g., usually at least the library of the pattern of neoplasm-specific marker detection results known to be indicative of the presence or absence of a pre-cancerous condition) are maintained on a server for access, e.g., confidential access. The results may be accessed or sent to professionals as desired.

A system for use in the methods described herein generally includes at least one computer processor (e.g., where the method is carried out in its entirety at a single site) or at least two networked computer processors (e.g., where detected marker data for a sample obtained from a subject is to be input by a user (e.g., a technician or someone performing the assays)) and transmitted to a remote site to a second computer processor for analysis (e.g., where the pattern of methylation-specific marker) detection results is compared to a library of patterns known to be indicative of the presence or absence of a pre-cancerous condition), where the first and second computer processors are connected by a network, e.g., via an intranet or internet). The system can also include a user component(s) for input; and a reviewer component(s) for review of data, and generation of reports, including detection of a pre-cancerous condition, staging and/or grading of a neoplasm, or monitoring the progression of a pre-cancerous condition or a neoplasm. Additional components of the system can include a server component(s); and a database(s) for storing data (e.g., as in a database of report elements, e.g., a library of marker patterns known to be indicative of the presence or absence of a pre-cancerous condition and/or known to be indicative of a grade and/or a stage of a neoplasm, or a relational database (RDB) which can include data input by the user and data output. The computer processors can be processors that are typically found in personal desktop computers (e.g., IBM, Dell, Macintosh), portable computers, mainframes, minicomputers, or other computing devices.

The input components can be complete, stand-alone personal computers offering a full range of power and features to run applications. The user component usually operates under any desired operating system and includes a communication element (e.g., a modem or other hardware for connecting to a network), one or more input devices (e.g., a keyboard, mouse, keypad, or other device used to transfer information or commands), a storage element (e.g., a hard drive or other computer-readable, computer-writable storage medium), and a display element (e.g., a monitor, television, LCD, LED, or other display device that conveys information to the user). The user enters input commands into the computer processor through an input device. Generally, the user interface is a graphical user interface (GUI) written for web browser applications.

The server component(s) can be a personal computer, a minicomputer, or a mainframe, or distributed across multiple servers (e.g., as in cloud computing applications) and offers data management, information sharing between clients, network administration and security. The application and any databases used can be on the same or different servers. Other computing arrangements for the user and server(s), including processing on a single machine such as a mainframe, a collection of machines, or other suitable configuration are contemplated. In general, the user and server machines work together to accomplish the processing of the present disclosure.

Where used, the database(s) is usually connected to the database server component and can be any device which will hold data. For example, the database can be any magnetic or optical storing device for a computer (e.g., CDROM, internal hard drive, tape drive). The database can be located remote to the server component (with access via a network, modem, etc.) or locally to the server component.

Where used in the system and methods, the database can be a relational database that is organized and accessed according to relationships between data items. The relational database is generally composed of a plurality of tables (entities). The rows of a table represent records (collections of information about separate items) and the columns represent fields (particular attributes of a record). In its simplest conception, the relational database is a collection of data entries that "relate" to each other through at least one common field.

Additional workstations equipped with computers and printers may be used at point of service to enter data and, in some embodiments, generate appropriate reports, if desired. The computer(s) can have a shortcut (e.g., on the desktop) to launch the application to facilitate initiation of data entry, transmission, analysis, report receipt, etc. as desired.

The following examples are provided in order to demonstrate and further illustrate certain preferred embodiments and aspects of the present invention and are not to be construed as limiting the scope thereof.

### Example 1

### Material and methods

### Urological cancer cell lines

Sixteen urological cancer cell lines, including eight from bladder- (5637, HT-1197, HT-1376, RT4, SW780, T-24, TCCSUP and UM-UC-3), four from renal- (786-O, ACHN, Caki-1 and Caki-2) and four from prostate cancer (DU 145, LNCaP, NCI-660 and PC-3) were obtained from the American Type Culture Collection. The bladder cancer cell lines, comprising the ATCC's Bladder Cancer Cell Panel (ATCC^{®} TCP-1020^{™}), were selected to represent varying degree of genetic complexity. All cell lines have been STR-tested and authenticated.

### Clinical samples

Sixty tissue samples were included in the present study, comprising 20 bladder cancer (BlCa), 10 renal cancer (ReCa), 10 prostate cancer (PrCa), and 20 normal bladder mucosa (NBM) samples. The BlCa, ReCa and NBM tissues were obtained from a consecutive series of patients diagnosed and treated at the Portuguese Oncology Institute - Porto, Portugal. The PrCa tissue was collected during prostatectomies undertaken at the Oslo University Hospital - Radiumhospitalet.

The urine samples were collected from patients undergoing cystoscopy at the Aker University Hospital from December 2016 to July 2017, as well as from self-declared healthy donors collected at the Norwegian Radium Hospital in May 2017. Collection of the Oslo series was initiated for the present study, and 26 bladder, 12 renal and 18 prostate cancer patients, as well as 30 healthy individuals, were included for analyses. For the large majority of cases, two independent urinates were analyzed. Details on the tissue and urine samples are summarized in Table 1.

### Reduced representation bisulfite sequencing (RRBS) data analysis

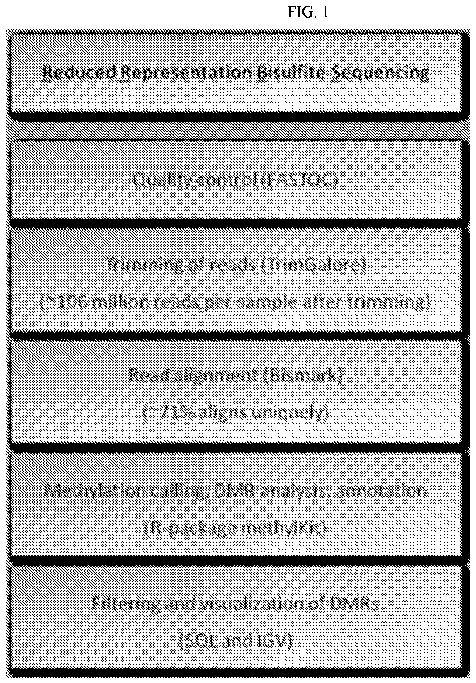

RRBS library construction and sequencing of the 16 urological cancer cell lines were performed at Beijing Genomics Institute (BGI) as previously described²⁵. The following bioinformatic pipeline was established in house (Figure 1): Quality control of the sequence reads was performed using FASTQC (version 0.10.1). Low quality reads and Illumina adapters were trimmed using the wrapper script TrimGalore (version 0.3.3), which trims reads using Cutadapt²⁶ and checks quality afterwards using FASTQC. Trimmed reads were aligned to a bisulfite converted version of the human genome (hg19) using Bismark (version 0.10.0)²⁷, and ran with Bowtie2²⁸. Samtools (version 0.1.18)²⁹ was employed to edit the alignment files. MethylKit (version 0.9.2)³⁰ was used to calculate the methylation status of each CpG site covered. Calculations of differentially methylated regions were performed across the different cancer types considering 'windows' of both 200 bp and 1000 bp, using a 100 bp step size between windows. Lists of differentially methylated regions were combined and filtered employing an SQL database (SQLite studio version 3.0.5). In addition to selecting a methylation difference over 25% between bladder cancer and renal/prostate cancer, regions displaying ≥50% methylation in at least 7/8 of the bladder cancer cell lines, and simultaneously less than 20% methylation in 6/8 of the renal- and prostate-cancer cell lines were considered biomarker candidates. Bismark methylation extractor was utilized to extract bedgraphs from aligned BAM files. The bedgraphs were used for visualization of regions of interest in Integrative Genomics Viewer (IGV, version 2.3)³¹.

### Urine processing, DNA isolation and bisulfite conversion

Urine samples were processed within 0-4 hours from sampling. Cells were collected by centrifugation. Centrifugation was performed at 3200 rcf at 4°C for 20 minutes, before the supernatant was replaced with PBS and centrifugation repeated. Cell pellets were stored at - 80°C, until DNA was isolated using the QIAamp DNA Mini Kit (Qiagen). Isolation of DNA from tissue, as well as from urological cell lines, has been described previously^{34,35}.

DNA concentrations were measured using the NanoDrop 1000 Spectrophotometer (Thermo Fisher Scientific). The EpiTect Bisulfite Kit (Qiagen) was used for bisulfite conversion, and samples were automatically purified and eluted by the QIAcube System (Qiagen). The DNA input was 1300 ng for cell lines and tissue and 430 ng for urine samples. All kits were used according to the manufacturers' standard protocols.

### Targeted DNA methylation analyses: quantitative methylation-specific PCR (qMSP) and droplet digital PCR (ddPCR)

DNA from cancer cell lines and tissue samples (32.5ng) was analyzed by qMSP using the 7900HT Real-Time PCR System (Life Technologies) as previously described³⁶. The repetitive element ALU-C4³⁷ was used for normalization. The percentage of methylated molecules per sample (PMR value) was calculated by dividing the normalized quantity (target/ALU-C4) of the samples by the normalized quantity of the methylation positive control (universal methylated human DNA standard; Zymo Research), and multiplying by 100. As a general rule, the highest PMR value in normal bladder mucosa was used as cutoff for positive methylation scoring, ensuring assay specificity. Urine DNA (32.5ng) was analyzed by droplet digital PCR (ddPCR) using the QX200^{™} Droplet Digital^{™} PCR System (BioRad) as previously described²³. The 4Plex was used for normalization, and the PoDCall algorithm for positive droplet calling²³. The ddPCR methylation concentrations were normalized by dividing the concentration of the target on the concentration of the 4Plex and multiplying by a constant of 400. For all analyses, universal methylated human DNA standard (Zymo Research) was used as methylation-positive control, normal blood of healthy donors and/or human WGA non-methylated DNA (Zymo Research) as methylation-negative controls, and RNase-free water (Sigma Aldrich) as non-template control (NTC). All assay primer and probe sequences are listed in Table 2.

### Results

### A novel bioinformatic pipeline for genome-wide identification of DNA methylation biomarkers

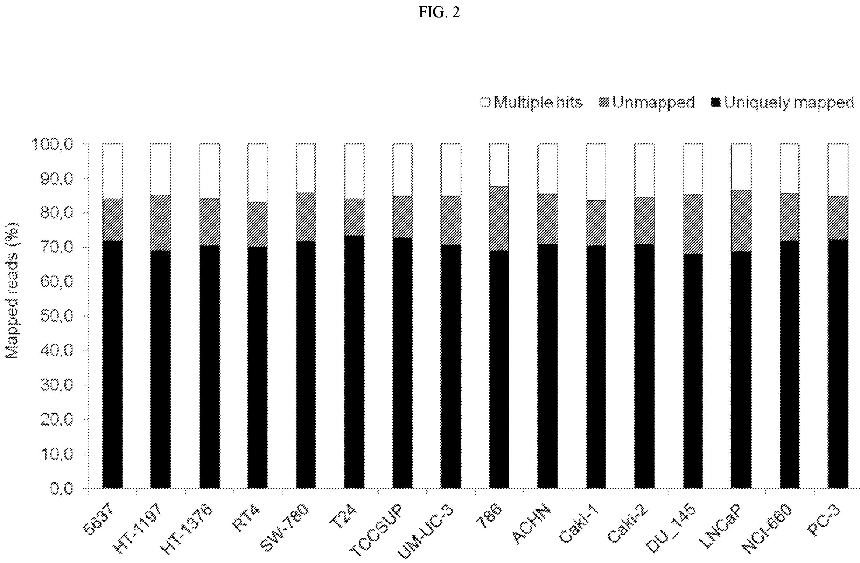

Approximately 106 million sequencing reads were left per sample after quality control and trimming. The average mapping efficiency was 70.9% uniquely mapped reads, 15.0% reads with multiple hits and 14.1% unmapped reads (Figure 2). Only uniquely mapped reads were kept for downstream analyses. All samples showed near complete bisulfite conversion (≥99.5%). The average coverage and methylation distribution per sample was 111.6X and 37.2%, respectively. There was a considerable variation in methylation level both between and within the different groups (bladder, prostate and renal cancer) which was largest for the bladder cancer group. Calculation and filtering of differentially methylated regions resulted in 214 windows fulfilling all selection criteria (described in Materials and Methods). These comprised 64 windows à 200 bp and 150 windows à 1000 bp, with a high degree of overlap. Overlapping windows were considered to represent the same biomarker candidate (see Figure 3 for a representative example), resulting in the identification of 32 candidates (Table 3). Notably, 59% (19/32) of the candidates were located more than 1500 bp away from the nearest transcription start site.

### Developing a digital PCR based test - targeted analyses of biomarker candidates

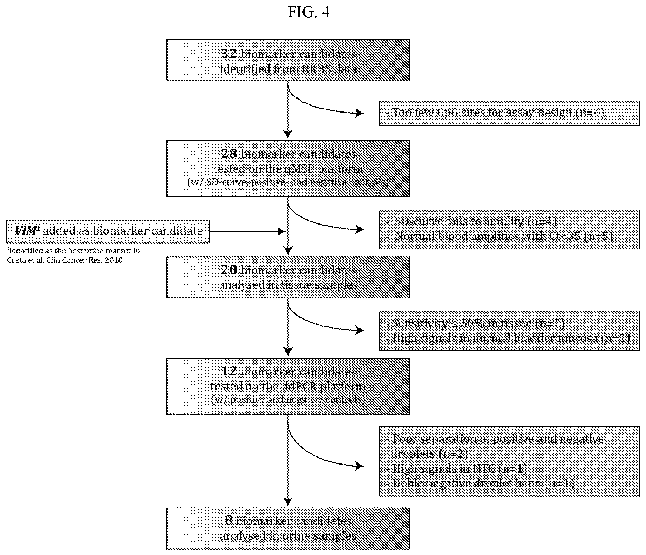

QMSP assays were designed for each of the 32 biomarker candidates. Nineteen of 32 assays passed the quality control (as outlined in Figure 4). All displayed high concordance with the RRBS data in the 16 urological cancer cell lines tested. Vimentin (*VIM*), identified as a promising bladder cancer DNA methylation biomarker from our previous study³⁴, was included in subsequent analyses. The performance of the resulting 20 biomarker candidates in tissue analyses is shown in Table 4. Sensitivities ranged from 25-100%, with a corresponding specificity of 100% for all markers (with the exception of 19-15288601). When including renal and prostate cancer tissue in the control group, the specificity of the markers remained high (90-100%; median=97.5%; Table 4). One biomarker candidate was discarded from further analyses due to high signals in normal bladder mucosa (17-38347601; Table 4). Biomarker candidates achieving >50% sensitivity in tissue analyses (n=12) were transferred to the ddPCR platform. Eight assays showed good technical performance on ddPCR (Figure 4), and were analyzed in urine samples.

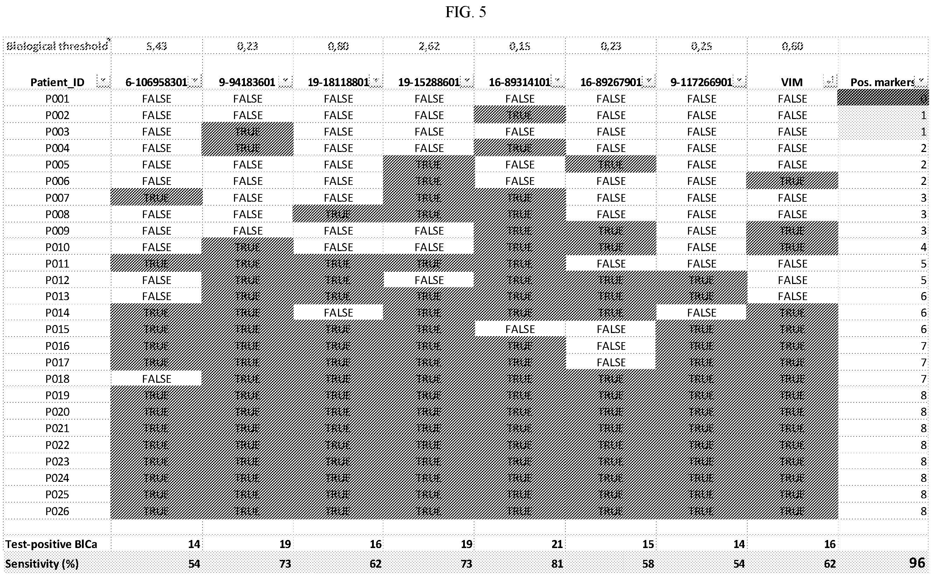

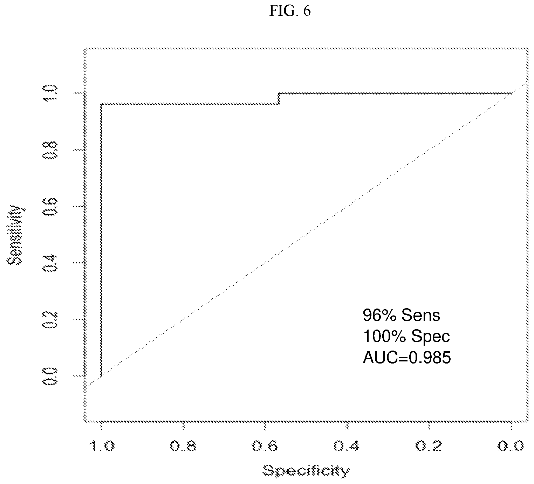

The individual diagnostic performances of the 8 markers in urine (at diagnostic thresholds ensuring 100% specificity for differentiating between bladder cancer and healthy controls), is illustrated in Figure 5 and summarized in Table 5. Notably, several of the new biomarkers exhibited higher diagnostic performance than the well-validated biomarker VIM, with the best new biomarker exhibiting 81% sensitivity vs 62% for VIM. All but one of the bladder cancer cases (25/26) could be detected by at least one marker, the vast majority of which (23/26) being positive for multiple markers. Combining multiple markers gradually increased sensitivity, with the best two marker combination achieving 94% sensitivity, while 3 markers were sufficient to achieve optimal (96%) sensitivity in this discovery cohort (Table 5). Receiver operating_characteristic (ROC) curve analysis for an illustrative 5-marker combination is shown Figure 6. Including renal and prostate cancer samples in the control group, the accuracy of the biomarkers remained high, with a resulting specificity of 80% (Table 5). This indicates that the markers are highly bladder cancer specific.

### Application of the biomarkers in urine-based monitoring of TURB-treated patients for disease recurrence

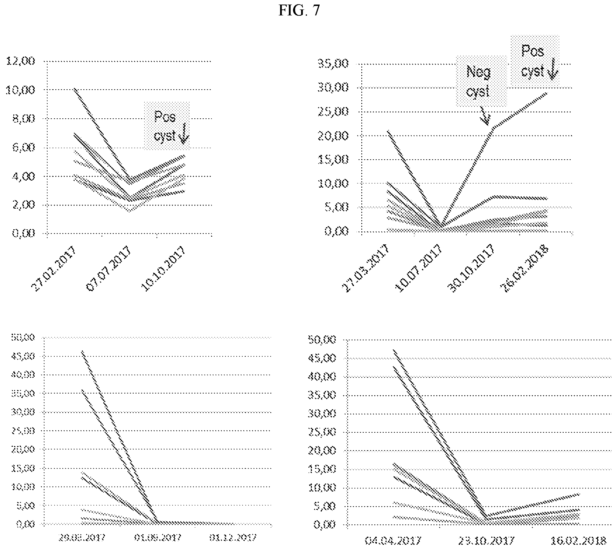

To evaluate the accuracy of our biomarker panel in detecting recurrences in a surveillance setting, a prospective clinical study has been initiated following 50 bladder cancer patients for two years, treated at Oslo University Hospital-Aker. Urine samples are collected before surgery and before every scheduled cystoscopy during the two-year follow-up period. The combination of test results from pre-surgery and first scheduled follow-up samples served as a baseline for diagnostic calls on disease recurrence. Generally, marker levels were dramatically reduced or abolished at the first follow-up compared to the pre-surgery test, while elevation of marker levels at subsequent follow-ups indicated disease recurrence. Preliminary data of urinary sample analysis of the 8 biomarkers from the first 2-4 follow-ups of 26 patients has been analyzed, with highly promising results. Eleven histology-confirmed recurrences (in 10 patients) from the second scheduled follow-up onwards were all detected by at least one of our urine-based biomarkers, with the majority of recurrences being indicated by multiple markers (illustrative example in Figure 7, top left). Interestingly, three of these recurring cancers were of the non-invasive Ta stage, which has been demonstrated to be difficult to detect by cytology³ and sometimes missed even by cystoscopy⁸.

The majority (12/18) of samples with no histologically confirmed recurrence, had negative test results for all our biomarkers (illustrative example shown in Figure 7, bottom left). Six samples had positive biomarker test results (illustrative example shown in Figure 7, bottom right) that could represent either false positives or early indications of recurrence, which would be expected to be confirmed by cystoscopy and histology at later follow-ups. This potential is indicated by the observation that in 5 out of 11 cases of confirmed recurrences, our test was able to identify the recurrence 3-4 months before cystoscopy (illustrative example in Figure 7, top right). This highlights the use of a test based on our biomarker panel as a reliable non-invasive early indicator of recurrence, reducing the need for more invasive and costly monitoring cystoscopies. In a clinical setting where a urine based test based on our markers is implemented in monitoring, a false positive test result would only trigger a cystoscopy evaluation (i.e. no change to current practice).

### Example 2

Altogether 60 patients with histologically confirmed non-muscle invasive bladder cancer (NMIBC) have been studied. All patients have been followed for at least 1 year, and 36 of the patients have been followed for 2 years (i.e. have reach the end of the study period). For each patient, there are from 1 to 8 surveillance cystoscopy points with matched clinical and molecular data.

Almost 300 cystoscopies have been performed altogether, among which 51 were positive. Only 24 of the positive cystoscopies were confirmed to be actual recurrences (confirmed by histology or based on tumor destruction), underscoring the lack of accuracy of cystoscopy. Among the 24 confirmed recurrences, 17 represent surveillance points where the cystoscopy in theory can be replaced (i.e. all surveillance points except the first after TURB or tumor destruction). The urine-based test described herein was positive in 16 of the cases, resulting in 94% sensitivity. For the remaining 7 recurrences (i.e. cystoscopies that cannot be replaced by non-invasive tests), our test was positive in 3 of the cases. Moreover, in 11 cases the urine-based test detected the recurrence earlier than cystoscopy, and in 4 cases the test was positive as early as 7-8 months before the recurrence was detected in the clinic.

The specificity of the test will be evaluated once all patients have reached the end of the study. As mentioned above, there are several examples of confirmed early detections in the existing data set, and there are still around 30 positive tests that indicate potential recurrences.

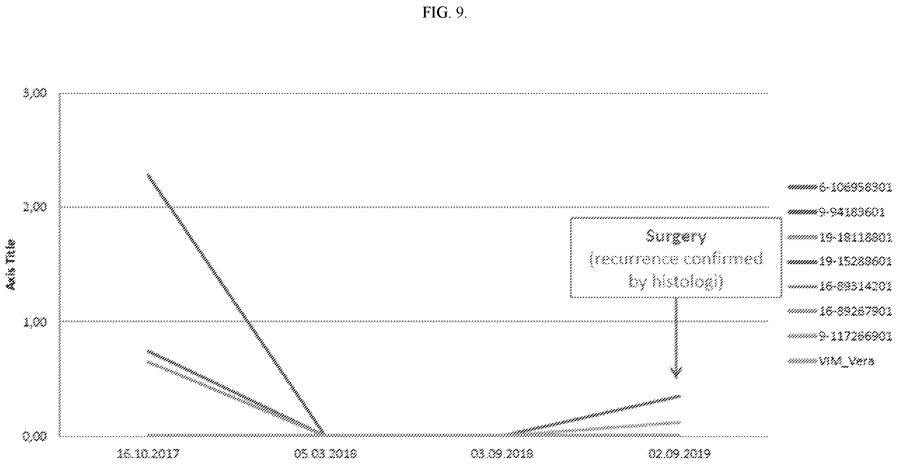

Data from exemplary cases are presented in FIGs. 9-13. Each plot represents the clinical course of one patient. Each colored line represents one biomarker (n=8 in total; see legend to the right). An increase in biomarker level is hypothesized to indicate recurrence. A decrease in biomarker level is expected after a clinical intervention where the tumor has been removed (surgery/tumor destruction). X-axis: time point of a clinical intervention (cystoscopy control/surgery/destruction of tumor with laser, etc.) and concomitant urine sampling. The first time point on each graph represents surgical removal of a tumor, with a corresponding urine sample taken before surgery (i.e. when the tumor was present). Y-axis: normalized biomarker concentration.

**Case 1: Patient with a clinically confirmed recurrence #1.** Biomarker levels drop after the initial surgery and no increase is seen at the follow up control 03.09.2018, where the cystoscopy is also negative. At the next follow up 02.09.2019, the biomarker levels have started to rice, and a recurrent tumor is confirmed in the clinic by histology. See FIG. 9.

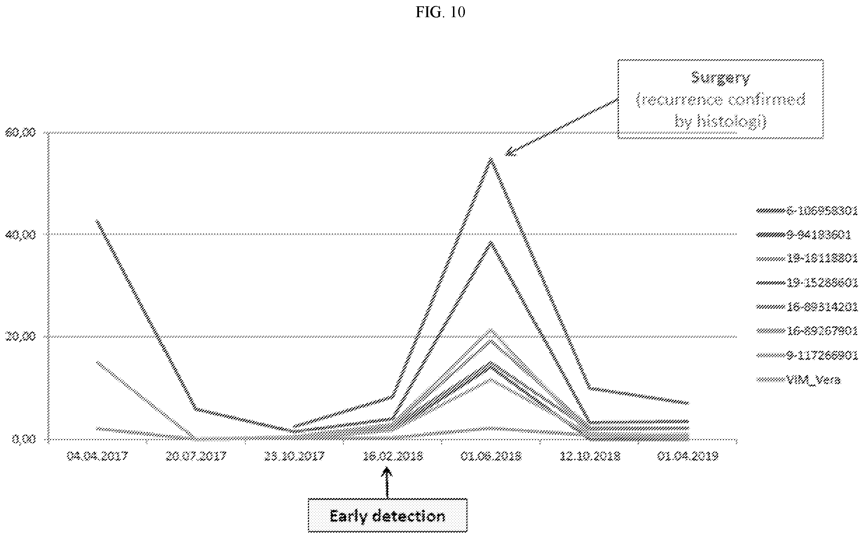

**Case 2: Patient with a clinically confirmed recurrence #2.** Biomarker levels drop after the initial surgery and continue to decrease. The 16.02.2018, the biomarker levels have started to rise, but the cystoscopy is negative. 4 months later (01.06.2018) the increase in biomarker concentrations continue, and a recurrent tumor is confirmed in the clinic by histology. At the first follow up control after surgery (12.10.2018), the biomarker concentrations decrease again. See FIG. 10.

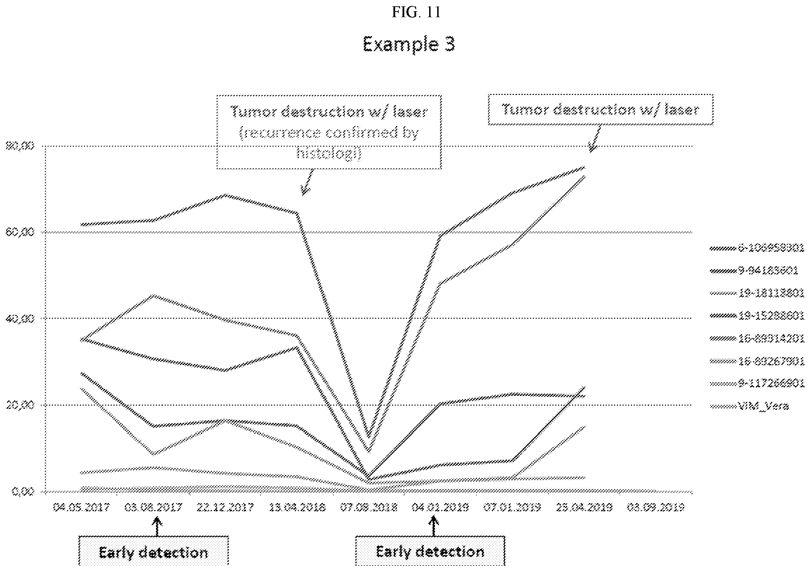

**Case 3: Patient with two clinically confirmed recurrences.** Biomarker levels do not drop as expected after initial surgery (could indicate incomplete removal of the tumor and a residual cancer). The 13.04.2017, a tumor is confirmed by histology and tumor destruction using laser is performed. The biomarker concentrations then drop, but subsequently start to rise again. The 23.04.2019, a tumor is detected and destroyed using laser. See FIG. 11.

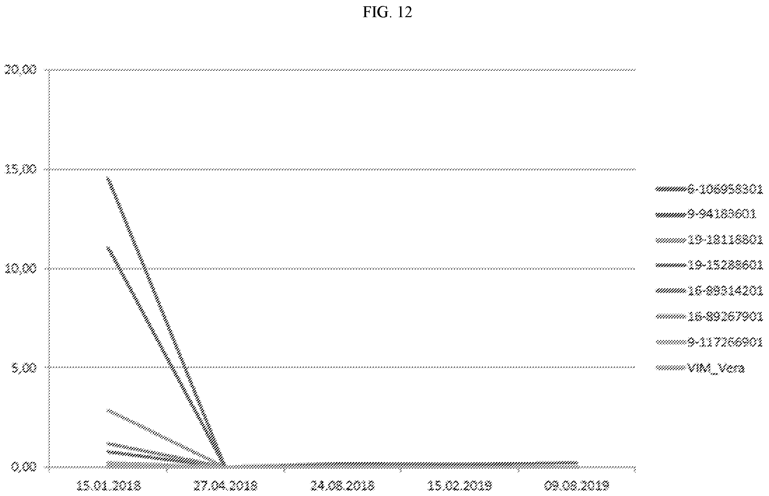

**Case 4: Patient without clinically confirmed recurrence #1.** Biomarker levels drop after the initial surgery, and stay low at all the subsequent follow up controls. The corresponding cystoscopies are negatives. See FIG. 12.

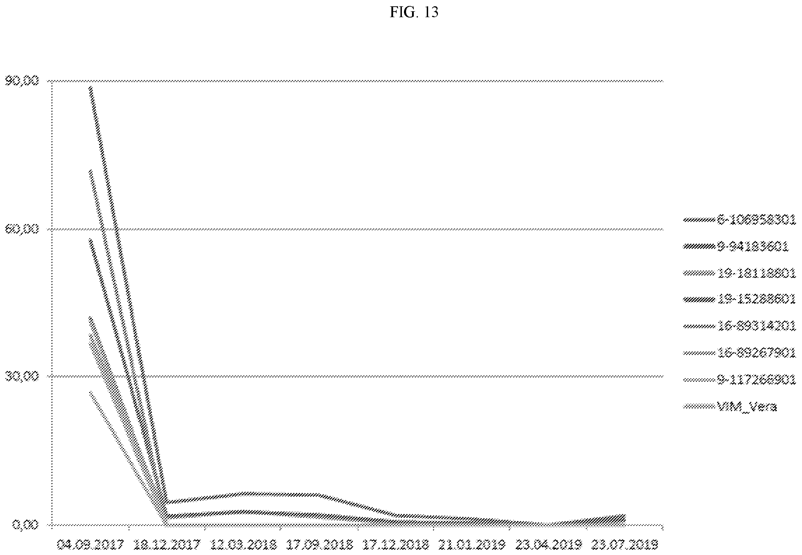

**Case 5: Patient without clinically confirmed recurrence #2.** Biomarker levels drop after the initial surgery, and continue to decrease with corresponding negative cystoscopies (except for a slight increase seen for 3 of the biomarkers 12.03.2018, *i.e.* false positives). The 23.07.2019, some of the biomarkers have started to increase, which could indicate a recurrent tumor. See FIG. 13.

### Reference List

**1.** Antoni S, Ferlay J, Soerjomataram I, Znaor A, Jemal A, Bray F. Bladder Cancer Incidence and Mortality: A Global Overview and Recent Trends. Eur Urol. Jun 28 2016.
**2.** Babjuk M, Bohle A, Burger M, et al. EAU Guidelines on Non-Muscle-invasive Urothelial Carcinoma of the Bladder: Update 2016. Eur Urol. Mar 2017;71(3):447-461.
**3.** Sun M, Trinh QD. Diagnosis and staging of bladder cancer. Hematol Oncol Clin North Am. Apr 2015;29(2):205-218, vii.
**4.** van den Bosch S, Alfred Witjes J. Long-term cancer-specific survival in patients with high-risk, non-muscle-invasive bladder cancer and tumour progression: a systematic review. Eur Urol. Sep 2011;60(3):493-500.
**5.** Svatek RS, Hollenbeck BK, Holmang S, et al. The economics of bladder cancer: costs and considerations of caring for this disease. Eur Urol. Aug 2014;66(2):253-262.
**6.** Lee CS, Yoon CY, Witjes JA. The past, present and future of cystoscopy: the fusion of cystoscopy and novel imaging technology. BJU International. 2008;102(9b):1228-1233.
**7.** Mowatt G, Zhu S, Kilonzo M, et al. Systematic review of the clinical effectiveness and cost-effectiveness of photodynamic diagnosis and urine biomarkers (FISH, ImmunoCyt, NMP22) and cytology for the detection and follow-up of bladder cancer. Health Technol Assess. Jan 2010;14(4):1-331, iii-iv.
**8.** Grossman HB, Gomella L, Fradet Y, et al. A phase III, multicenter comparison of hexaminolevulinate fluorescence cystoscopy and white light cystoscopy for the detection of superficial papillary lesions in patients with bladder cancer. J Urol. Jul 2007;178(1):62-67.
**9.** Witjes JA, Douglass J. The role of hexaminolevulinate fluorescence cystoscopy in bladder cancer. Nat Clin Pract Urol. Oct 2007;4(10):542-549.
**10.** Knowles MA, Hurst CD. Molecular biology of bladder cancer: new insights into pathogenesis and clinical diversity. Nat Rev Cancer. Jan 2015;15(1):25-41.
**11.** Lin SY, Linehan JA, Wilson TG, Hoon DSB. Emerging Utility of Urinary Cell-free Nucleic Acid Biomarkers for Prostate, Bladder, and Renal Cancers. Eur Urol Focus. Apr 2017;3(2-3):265-272.
**12.** Li HT, Duymich CE, Weisenberger DJ, Liang G. Genetic and Epigenetic Alterations in Bladder Cancer. Int Neurourol J. Nov 2016;20(Suppl 2):S84-94.
**13.** Chou R, Gore JL, Buckley D, et al. Urinary Biomarkers for Diagnosis of Bladder Cancer: A Systematic Review and Meta-analysis. Ann Intern Med. Dec 15 2015;163(12):922-931.
**14.** Maas M, Walz S, Stuhler V, et al. Molecular markers in disease detection and follow-up of patients with non-muscle invasive bladder cancer. Expert Rev Mol Diagn. May 2018;18(5):443-455.
**15.** Bosschieter J, Lutz C, Segerink LI, et al. The diagnostic accuracy of methylation markers in urine for the detection of bladder cancer: a systematic review. Epigenomics. Apr 25 2018.
**16.** D'Costa JJ, Goldsmith JC, Wilson JS, Bryan RT, Ward DG. A Systematic Review of the Diagnostic and Prognostic Value of Urinary Protein Biomarkers in Urothelial Bladder Cancer. Bladder Cancer. Jul 27 2016;2(3):301-317.
**17.** Di Meo A, Bartlett J, Cheng Y, Pasic MD, Yousef GM. Liquid biopsy: a step forward towards precision medicine in urologic malignancies. Mol Cancer. Apr 14 2017;16(1):80.
**18.** Sapre N, Anderson PD, Costello AJ, Hovens CM, Corcoran NM. Gene-based urinary biomarkers for bladder cancer: an unfulfilled promise? Urol Oncol. Jan 2014;32(1):48 e49-17.
**19.** Kandimalla R, van Tilborg AA, Zwarthoff EC. DNA methylation-based biomarkers in bladder cancer. Nat.Rev. Urol. 6/2013 2013;10(6):327-335.
**20.** Koch A, Joosten SC, Feng Z, et al. Analysis of DNA methylation in cancer: location revisited. Nat Rev Clin Oncol. Apr 17 2018.
**21.** Schulz WA, Goering W. DNA methylation in urothelial carcinoma. Epigenomics. Oct 2016;8(10):1415-1428.
**22.** Meissner A, Gnirke A, Bell GW, Ramsahoye B, Lander ES, Jaenisch R. Reduced representation bisulfite sequencing for comparative high-resolution DNA methylation analysis. Nucleic Acids Res. 2005;33(18):5868-5877.
**23.** Pharo HD, Andresen K, Berg KCG, Lothe RA, Jeanmougin M, Lind GE. A robust internal control for high-precision DNA methylation analyses by droplet digital PCR. Clin Epigenetics. 2018;10:24.
**24.** Postel M, Roosen A, Laurent-Puig P, Taly V, Wang-Renault SF. Droplet-based digital PCR and next generation sequencing for monitoring circulating tumor DNA: a cancer diagnostic perspective. Expert Rev Mol Diagn. Nov 13 2017:1-11.
**25.** Wang L, Sun J, Wu H, et al. Systematic assessment of reduced representation bisulfite sequencing to human blood samples: A promising method for large-sample-scale epigenomic studies. J Biotechnol. Jan 2012;157(1):1-6.
**26.** Martin M. Cutadapt removes adapter sequences from high-throughput sequencing reads. EMBnet.journal. 2011;17(1):10-12.
**27.** Krueger F, Andrews SR. Bismark: a flexible aligner and methylation caller for Bisulfite-Seq applications. Bioinformatics. Jun 1 2011;27(11):1571-1572.
**28.** Langmead B, Salzberg SL. Fast gapped-read alignment with Bowtie 2. Nat Methods. Mar 4 2012;9(4):357-359.
**29.** Li H, Handsaker B, Wysoker A, et al. The Sequence Alignment/Map format and SAMtools. Bioinformatics. Aug 15 2009;25(16):2078-2079.
**30.** Akalin A, Kormaksson M, Li S, et al. methylKit: a comprehensive R package for the analysis of genome-wide DNA methylation profiles. Genome Biol. Oct 3 2012;13(10):R87.
**31.** Robinson JT, Thorvaldsdottir H, Winckler W, et al. Integrative genomics viewer. Nat Biotechnol. Jan 2011;29(1):24-26.
**32.** Andersson E, Dahmcke CM, Steven K, Larsen LK, Guldberg P. Filtration Device for On-Site Collection, Storage and Shipment of Cells from Urine and Its Application to DNA-Based Detection of Bladder Cancer. PLoS One. 2015;10(7):e0131889.
**33.** Dahmcke CM, Steven KE, Larsen LK, et al. A Prospective Blinded Evaluation of Urine-DNA Testing for Detection of Urothelial Bladder Carcinoma in Patients with Gross Hematuria. Eur Urol. Dec 2016;70(6):916-919.
**34.** Costa VL, Henrique R, Danielsen SA, et al. Three epigenetic biomarkers, GDF15, TMEFF2, and VIM, accurately predict bladder cancer from DNA-based analyses of urine samples. Clin Cancer Res. Dec 01 2010;16(23):5842-5851.
**35.** Lovf M, Zhao S, Axcrona U, et al. Multifocal Primary Prostate Cancer Exhibits High Degree of Genomic Heterogeneity. Eur Urol. Sep 1 2018.
**36.** Andresen K, Boberg KM, Vedeld HM, et al. Novel target genes and a valid biomarker panel identified for cholangiocarcinoma. Epigenetics. 11/2012 2012;7(11):1249-1257.
**37.** Weisenberger DJ, Campan M, Long TI, et al. Analysis of repetitive element DNA methylation by MethyLight. Nucleic Acids Res. 2005 2005;33(21):6823-6836.
**38.** Yossepowitch O, Herr HW, Donat SM. Use of urinary biomarkers for bladder cancer surveillance: patient perspectives. J Urol. Apr 2007;177(4):1277-1282; discussion 1282.
**39.** Bennett CW, Berchem G, Kim YJ, El-Khoury V. Cell-free DNA and next-generation sequencing in the service of personalized medicine for lung cancer. Oncotarget. 2016;7(43):71013-71035.
**40.** Griffith P, Sun D, Tritsch S, et al. Droplet-Digital PCR Provides a Rapid, Accurate and Cost-Effective Method for Identification of Biomarker FcγRIIIa-F158V Genotypes. Gene Technol Sep 20, 2017 2017;6(143).
**41.** Leygo C, Williams M, Jin HC, et al. DNA Methylation as a Noninvasive Epigenetic Biomarker for the Detection of Cancer. Dis Markers. 2017;2017:3726595.
**42.** Brait M, Begum S, Carvalho AL, et al. Aberrant promoter methylation of multiple genes during pathogenesis of bladder cancer. Cancer Epidemiol Biomarkers Prev. Oct 2008; 17(10):2786-2794.

## Claims

1. A method of identifying methylation markers in a sample, comprising: detecting the presence of aberrant methylation in nucleic acid SEQ ID NO: 1, and one or more nucleic acids selected from the group consisting of SEQ ID NOs: 2-7 in a sample from a subject.

2. The method of claim 1, wherein said sample is urine.

3. The method of claim 1 or 2, wherein said subject has previously been treated for bladder cancer, preferably wherein said treatment is trans urethreal transection of bladder tumor TURBT, or wherein said subject is suspected of having bladder cancer; preferably wherein said bladder cancer is non-muscle invasive bladder cancer.

4. The method of claim 1 to 3, wherein said methylation is detected by a method selected from the group consisting of methylation specific PCR, digital droplet PCR, methylated DNA immunoprecipitation (MeDIP), and pyrosequencing of bisulfite treated DNA.

5. The method of any one of claims 1 to 4, wherein said one or more nucleic acids are four or more.

6. The method of any one of claims 1 to 4, wherein said one or more nucleic acids are all of said nucleic acids.

7. The method of any one of claims 1 to 6, wherein the method comprises detecting the presence of aberrant methylation in nucleic acid SEQ ID NO: 15, combined with one or more nucleic acids selected from the group consisting of SEQ ID NOs: 16-21.

8. The method of any one of claims 1 to 7, wherein said aberrant methylation is a change in methylation levels or patterns relative to a control nucleic acid, preferably wherein said control nucleic acid is an unmethylated nucleic acid or a nucleic acid from a subject not diagnosed with bladder cancer.

9. The method of any one of claims 1 to 8, further comprising detecting the presence of aberrant methylation in a VIM nucleic acid.

10. The method of any one of claims 1 to 9, wherein said detecting comprises the steps of a) modifying said nucleic acid with bisulfite; and b) detecting said bisulfite modified nucleic acid using an amplification method.

11. Method for selecting a subject for treatment with chemotherapy, radiation or surgery to thereby treat recurrent bladder cancer, wherein the method comprises:
a) detecting the presence of aberrant methylation in nucleic acid SEQ ID NO: 1 and one or more nucleic acids selected from the group consisting of SEQ ID Nos: 2-7 nucleic acids in a urine sample from a subject previously treated for bladder cancer using the method of any one of claims 1 to 10;
b) identifying a subject with said aberrant methylation; and
c) selecting the subject for treatment.

12. A method of diagnosing bladder cancer, comprising:
a) identifying the presence of aberrant methylation in a nucleic acid in a sample from a subject as defined in any one of claims 1 to 10; and
b) diagnosing bladder cancer in said subject when said methylation is present.

13. A kit, comprising:
a plurality of nucleic acid reagents that detect the presence of aberrant methylation in nucleic acid SEQ ID NO: 1, and one or more nucleic acids selected from the group consisting of SEQ ID NOs: 2-7.

14. The kit according to claim 13, wherein said reagents are nucleic acid primers or probes that bind to bisulfite modified nucleic acids but not unmodified nucleic acids, preferably wherein said reagents bind to nucleic acid SEQ ID NO: 8 and one or more nucleic acids selected from the group consisting of SEQ ID NOs: 9-14 or bind to nucleic acid SEQ ID NO: 22 and one or more nucleic acids selected from the group consisting of SEQ ID NO: 23-28.

15. The kit according to claim 13 or 14, wherein said nucleic acid reagents further detect the presence of aberrant methylation in a VIM nucleic acid.

## Patentansprüche

1. Verfahren zur Identifizierung von Methylierungsmarkern in einer Probe, umfassend: Nachweisen des Vorhandenseins von aberranter Methylierung in der Nukleinsäure SEQ ID NO: 1 und einer oder mehreren Nukleinsäuren, ausgewählt aus der Gruppe bestehend aus SEQ ID NOs: 2-7, in einer Probe aus einem Subjekt.

2. Verfahren nach Anspruch 1, wobei es sich bei der Probe um Urin handelt.

3. Verfahren nach Anspruch 1 oder 2, wobei das Subjekt zuvor wegen Blasenkrebs behandelt wurde, wobei es sich vorzugsweise bei besagter Behandlung um transurethrale Transsektion des Blasentumors TURBT handelt, oder wobei das Subjekt im Verdacht steht, Blasenkrebs aufzuweisen; vorzugsweise wobei der Blasenkrebs ein nicht-muskelinvasiver Blasenkrebs ist.

4. Verfahren nach Anspruch 1 bis 3, wobei die Methylierung durch ein Verfahren, ausgewählt aus der Gruppe bestehend aus Methylierungs-spezifischer PCR, digitaler Tröpfchen-PCR, Immunpräzipitation methylierter DNA (MeDIP) und Pyrosequenzierung Bisulfit-behandelter DNA, nachgewiesen wird.

5. Verfahren nach einem der Ansprüche 1 bis 4, wobei es sich bei der einen oder den mehreren Nukleinsäuren um vier oder mehr handelt.

6. Verfahren nach einem der Ansprüche 1 bis 4, wobei es sich bei der einen oder den mehreren Nukleinsäuren um alle von den Nukleinsäuren handelt.

7. Verfahren nach einem der Ansprüche 1 bis 6, wobei das Verfahren das Nachweisen des Vorhandenseins von aberranter Methylierung in der Nukleinsäure SEQ ID NO: 15, kombiniert mit einer oder mehreren Nukleinsäuren, ausgewählt aus der Gruppe bestehend aus SEQ ID NOs: 16-21, umfasst.

8. Verfahren nach einem der Ansprüche 1 bis 7, wobei es sich bei der aberranten Methylierung um eine Änderung der Methylierungsniveaus oder -muster relativ zu einer Kontrollnukleinsäure handelt, vorzugsweise wobei die Kontrollnukleinsäure eine unmethylierte Nukleinsäure oder eine Nukleinsäure aus einem Subjekt ist, bei dem kein Blasenkrebs diagnostiziert wurde.

9. Verfahren nach einem der Ansprüche 1 bis 8, ferner umfassend das Nachweisen des Vorhandenseins von aberranter Methylierung in einer VIM-Nukleinsäure.

10. Verfahren nach einem der Ansprüche 1 bis 9, wobei das Nachweisen die Schritte des a) Modifizierens der Nukleinsäure mit Bisulfit; und b) Nachweisens der Bisulfit-modifizierten Nukleinsäure unter Verwendung eines Amplifikationsverfahrens umfasst.

11. Verfahren zum Auswählen eines Subjekts zur Behandlung mit Chemotherapie, Bestrahlung oder chiurgischer Operation, um dadurch rezidivierenden Blasenkrebs zu behandeln, wobei das Verfahren umfasst:
a) Nachweisen des Vorhandenseins von aberranter Methylierung in der Nukleinsäure SEQ ID NO: 1 und einer oder mehreren Nukleinsäuren, ausgewählt aus der Gruppe bestehend aus Nukleinsäuren der SEQ ID NOs: 2-7, in einer Urinprobe aus einem Subjekt, das zuvor wegen Blasenkrebs behandelt wurde, unter Verwendung des Verfahrens nach einem der Ansprüche 1 bis 10;
b) Identifizieren eines Subjekts mit der aberranten Methylierung; und
c) Auswählen des Subjekts zur Behandlung.

12. Verfahren zum Diagnostizieren von Blasenkrebs, umfassend:
a) Identifizieren des Vorhandenseins von aberranter Methylierung in einer Nukleinsäure in einer Probe aus einem Subjekt, wie in einem der Ansprüche 1 bis 10 definiert; und
b) Diagnostizieren von Blasenkrebs in dem Subjekt, wenn die Methylierung vorhanden ist.

13. Kit, umfassend:
eine Vielzahl von Nukleinsäurereagenzien, die das Vorhandensein von aberranter Methylierung in der Nukleinsäure SEQ ID NO: 1 und einer oder mehreren Nukleinsäuren, ausgewählt aus der Gruppe bestehend aus SEQ ID NOs: 2-7, nachweisen.

14. Kit gemäß Anspruch 13, wobei es sich bei den Reagenzien um Nukleinsäureprimer oder -sonden handelt, die an Bisulfit-modifizierte Nukleinsäuren aber nicht an unmodifizierte Nukleinsäuren binden, vorzugsweise wobei die Reagenzien binden an die Nukleinsäure SEQ ID NO: 8 und eine oder mehrere Nukleinsäuren, ausgewählt aus der Gruppe bestehend aus SEQ ID NOs: 9-14, oder binden an die Nukleinsäure SEQ ID NO: 22 und eine oder mehrere Nukleinsäuren, ausgewählt aus der Gruppe bestehend aus SEQ ID NO: 23-28.

15. Kit gemäß Anspruch 13 oder 14, wobei die Nukleinsäurereagenzien ferner das Vorhandensein von aberranter Methylierung in einer VIM-Nukleinsäure nachweisen.

## Revendications

1. Procédé d'identification de marqueurs de méthylation dans un échantillon, comprenant : la détection de la présence d'une méthylation aberrante dans l'acide nucléique de N° ID SEQ : 1, et dans un ou plusieurs acides nucléiques choisis dans le groupe constitué par les N^{os} ID SEQ : 2 à 7 dans un échantillon provenant d'un sujet.

2. Procédé de la revendication 1, dans lequel ledit échantillon est de l'urine.

3. Procédé de la revendication 1 ou 2, dans lequel ledit sujet a déjà été traité pour un cancer de la vessie, de préférence dans lequel ledit traitement est une résection transurétrale de tumeur de la vessie, RTUTV, ou dans lequel ledit sujet est suspecté d'être atteint d'un cancer de la vessie ; de préférence dans lequel ledit cancer de la vessie est un cancer de la vessie non-musculo-invasif.

4. Procédé des revendications 1 à 3, dans lequel ladite méthylation est détectée par un procédé choisi dans le groupe constitué par la PCR spécifique à la méthylation, la PCR digitale en gouttelettes, l'immunoprécipitation d'ADN méthylé (MeDIP) et le pyroséquençage de l'ADN traité au bisulfite.

5. Procédé de l'une quelconque des revendications 1 à 4, dans lequel le ou les plusieurs acides nucléiques sont au nombre de quatre ou plus.

6. Procédé de l'une quelconque des revendications 1 à 4, dans lequel le ou les plusieurs acides nucléiques représentent la totalité desdits acides nucléiques.

7. Procédé de l'une quelconque des revendications 1 à 6, dans lequel le procédé comprend la détection de la présence d'une méthylation aberrante dans l'acide nucléique de N° ID SEQ : 15, en combinaison avec un ou plusieurs acides nucléiques choisis dans le groupe constitué par les N° ID SEQ : 16 à 21.

8. Procédé de l'une quelconque des revendications 1 à 7, dans lequel ladite méthylation aberrante est un changement des profils ou des niveaux de méthylation par rapport à un acide nucléique témoin, de préférence dans lequel ledit acide nucléique témoin est un acide nucléique non méthylé ou un acide nucléique provenant d'un sujet non diagnostiqué avec un cancer de la vessie.

9. Procédé de l'une quelconque des revendications 1 à 8, comprenant en outre la détection de la présence d'une méthylation aberrante dans un acide nucléique associé au gène codant pour la vimentine, VIM.

10. Procédé de l'une quelconque des revendications 1 à 9, dans lequel ladite détection comprend les étapes de :
a) modification dudit acide nucléique par du bisulfite ; et b) détection dudit acide nucléique modifié par le bisulfite à l'aide d'un procédé d'amplification.

11. Procédé de sélection d'un sujet pour un traitement par chimiothérapie, radiothérapie ou chirurgie afin de traiter un cancer récurrent de la vessie, dans lequel le procédé comprend :
a) la détection de la présence d'une méthylation aberrante dans l'acide nucléique de N° ID SEQ : 1 et dans un ou plusieurs acides nucléiques choisis dans le groupe constitué par les acides nucléiques de N^{os} ID SEQ : 2 à 7 dans un échantillon d'urine provenant d'un sujet précédemment traité pour un cancer de la vessie à l'aide du procédé de l'une quelconque des revendications 1 à 10 ;
b) l'identification d'un sujet présentant ladite méthylation aberrante ; et
c) la sélection du sujet en vue du traitement.

12. Procédé de diagnostic du cancer de la vessie, comprenant :
a) l'identification de la présence d'une méthylation aberrante dans un acide nucléique dans un échantillon provenant d'un sujet tel que défini dans l'une quelconque des revendications 1 à 10 ; et
b) le diagnostic d'un cancer de la vessie chez ledit sujet lorsque ladite méthylation est présente.

13. Kit comprenant :
une pluralité de réactifs d'acide nucléique qui détectent la présence d'une méthylation aberrante dans l'acide nucléique de N° ID SEQ : 1, et dans un ou plusieurs acides nucléiques choisis dans le groupe constitué par les N^{os} ID SEQ : 2 à 7.

14. Kit selon la revendication 13, dans lequel lesdits réactifs sont des amorces ou des sondes d'acide nucléique qui se lient à des acides nucléiques modifiés par le bisulfite mais pas à des acides nucléiques non modifiés, de préférence dans lequel lesdits réactifs se lient à l'acide nucléique de N° ID SEQ : 8 et à un ou plusieurs acides nucléiques choisis dans le groupe constitué par les N^{os} ID SEQ : 9 à 14, ou se lient à l'acide nucléique de N° ID SEQ : 22 et à un ou plusieurs acides nucléiques choisis dans le groupe constitué par les N^{os} ID SEQ : 23 à 28.

15. Kit selon la revendication 13 ou 14, dans lequel lesdits réactifs d'acide nucléique détectent en outre la présence d'une méthylation aberrante dans un acide nucléique associé au gène codant pour la vimentine, VIM.
